(19) **Europäisches Patentamt**
**European Patent Office**
**Office européen des brevets**

(11) **EP 4 221 814 B1**

(12) **EUROPÄISCHE PATENTSCHRIFT**

(45) Veröffentlichungstag und Bekanntmachung des Hinweises auf die Patenterteilung:
**02.04.2025 Patentblatt 2025/14**

(21) Anmeldenummer: **21783537.0**

(22) Anmeldetag: **01.10.2021**

(51) Internationale Patentklassifikation (IPC):
*A61N 1/05* *(2006.01)* *A61N 1/36* *(2006.01)*
*A61N 1/362* *(2006.01)*

(52) Gemeinsame Patentklassifikation (CPC):
**A61N 1/05;** A61N 1/0541; A61N 1/0565;
A61N 1/36038; A61N 1/362

(86) Internationale Anmeldenummer:
**PCT/EP2021/077108**

(87) Internationale Veröffentlichungsnummer:
**WO 2022/069713 (07.04.2022 Gazette 2022/14)**

(54) **AKTIVES IMPLANTIERBARES MEDIZINPRODUKT UND VERFAHREN ZU DESSEN HERSTELLUNG**

ACTIVE IMPLANTABLE MEDICAL PRODUCT AND METHOD FOR PRODUCING SAME

DISPOSITIF MÉDICAL IMPLANTABLE ACTIF ET SON PROCÉDÉ DE FABRICATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.10.2020 DE 102020212509**

(43) Veröffentlichungstag der Anmeldung:
**09.08.2023 Patentblatt 2023/32**

(73) Patentinhaber:
• **Johannes Kepler Universität Linz**
**4040 Linz (AT)**
• **NÖ Landesgesundheitsagentur**
**3100 St. Pölten (AT)**

(72) Erfinder:
• **HASSEL, Achim**
**4040 Linz (AT)**

• **KOLLENDER, Jan Philipp**
**4040 Linz (AT)**
• **DOLL, Theodor**
**80686 München (DE)**
• **SPRINZL, Georg Matthias**
**3100 St. Pölten (AT)**

(74) Vertreter: **Altmann Stößel Dick Patentanwälte PartG mbB**
**Theodor-Heuss-Anlage 2**
**68165 Mannheim (DE)**

(56) Entgegenhaltungen:
EP-A2- 1 421 972    WO-A1-2016/130402
WO-A1-2019/206664    US-A- 5 833 714
US-A1- 2009 240 296    US-A1- 2011 014 399

**Beschreibung**

Gebiet der Erfindung

Die vorliegende Erfindung betrifft ein aktives implantierbares Medizinprodukt und ein Verfahren zu dessen Herstellung. Das vorgeschlagene Medizinprodukt eignet sich insbesondere zur Verwendung in einem Cochlea Implantat oder als Herzschrittmacher; weitere Anwendungen sind denkbar.

Stand der Technik

Aus dem Stand der Technik sind aktive implantierbare Medizinprodukte, insbesondere für den Einsatz als Cochlea Implantat oder Herzschrittmacher, sowie Verfahren zu deren Herstellung bekannt. In Anlehnung an die Verordnung (EU) 2017/745 vom 5. April 2017, Kap. I, Art. 2 werden nachfolgend die folgenden Begriffsbestimmungen verwendet. Der Begriff "Medizinprodukt" bezeichnet eine Vorrichtung, die für einen medizinischen Zweck im oder am menschlichen oder tierischen Körper eingerichtet ist, insbesondere zur Behandlung oder Linderung einer Krankheit, jedoch auch zu deren Diagnose, Verhütung, Überwachung, Vorhersage oder Prognose, wobei eine bestimmungsgemäße Hauptwirkung im oder am Körper weder durch pharmakologische noch durch immunologische Mittel noch metabolisch erreicht wird.

Das Medizinprodukt wird dann als "aktives Medizinprodukt" bezeichnet, wenn für dessen Betrieb eine Energiequelle, ausgenommen der für diesen Zweck durch den Körper selbst oder durch Schwerkraft erzeugte Energie eingesetzt wird. Das Medizinprodukt wird mit dem Begriff "implantierbar" bezeichnet, wenn es dazu bestimmt ist, zumindest teilweise, d.h. ganz oder teilweise, in den Körper eingeführt zu werden, unabhängig davon, ob es dort verbleibt und/oder vollständig oder teilweise durch den Körper resorbierbar ist.

Insbesondere ist das aktive implantierbare Medizinprodukt ausgewählt aus der Gruppe umfassend Cochlea-Implantate, Retina-Implantate, implantierbare Kardioverter-Defibrillatoren, Blasen-Implantate zur Überwindung von Inkontinenz oder Implantate zur Tiefenhirn-Stimulation. Weitere Arten von aktiven implantierbaren Medizinprodukten sind jedoch möglich.

Die genannten aktiven implantierbaren Medizinprodukte zeichnen sich insbesondere dadurch aus, dass sie mindestens eine elektrisch leitfähige Elektrode aufweisen, die zur Stimulation einer zumindest teilweise der Elektrode zugewandten Gewebeumgebung im Körper, bevorzugt dort befindlicher Nerven oder Muskeln, eingerichtet ist. Zum Beispiel dient eine durch einen Herzschrittmacher umfasste Stimulationselektrode bestimmungsgemäß zur Anregung des Herzmuskels, während die Stimulationselektrode in einem Retina- oder Cochlea-Implantat bestimmungsgemäß die Nerven und/oder Ganglien der Sehbahn oder der Hörbahn beaufschlagen.

Trotz unterschiedlicher Formen verwenden die in den bekannten aktiven implantierbaren Medizinprodukten eingesetzten Stimulationselektroden üblicherweise ein auch als "Pt/Ir 90/10" bezeichnetes Elektrodenmaterial, das Platin (Pt) oder eine Platinlegierung, insbesondere Pt mit einer Beimischung von bevorzugt ca. 10% Iridium (Ir), umfasst. Dieser Einsatz dieses Elektrodenmaterials erfolgt weitgehend unverändert seit etwa 1970, obwohl es Anzeichen dafür gibt, siehe z.B. A. W. Hassel, J. P. Kollender, G. Sprinzl und T. Doll, Corrosion of active implant materials for cochlear hearing aids and cardiac pacemakers, EUROCORR 2017 & 20th ICC Prag, September 3-7, 2017, dass unter Betriebsbedingungen bei der Stimulation der Gewebeumgebung der Elektroden, insbesondere der hieran angrenzenden Nerven oder Muskeln, eine hohe elektrische Polarisierung auftritt, die zur Bildung von zusätzlichem Platinoxid führt.

Die Auflösung von Platinelektroden unter elektrischen, neurophysiologischen Stimulationsbedingungen wurde schon sehr früh beschrieben. Clark, Graeme, Cochlear Implants: Fundamentals and Applications, Springer 2003, ISBN 0-387-95583-6, Seite 171, gibt als Grenzwert $2\ mA/mm^2$ an, welche bei einer Repetitionsrate von 1 kHz bereits 20 ng Pt pro Tag in Lösung bringen können. Dabei gebildetes Platinoxid, das insbesondere zusammen mit bereits auf der Oberfläche der Elektrode vorhandenem Platinoxid während eines kathodischen Pulses delaminieren kann und damit elektrisch entkoppelt wird, kann somit chemisch zu metallischem Pt oder zu anderen Folgeprodukten reagieren, woraufhin ototoxische Effekte auftreten können, die eine Resthörfähigkeit des Patienten zerstören können.

Dieser Effekt ist darauf zurückzuführen, dass nicht nur völlig ertaubte Patienten, sondern auch über ein mit Restgehör insbesondere im Tieftonbereich verfügende Patienten mit einem Cochlea-Implantat ausgestattet werden. Basierend auf der Beobachtung, dass ein merklicher Teil dieser Patienten ihr Restgehör schneller verlieren, als dies bei progredientem Gehörsverlust zu erwarten wäre, auf der Kenntnis der oben beschriebenen Korrosion des Platins und auf dem Wissen um eine ototoxische Wirkung von cis-Platin, kann angenommen werden, dass von der Stimulationselektrode weg korrodiertes ionisches Pt dazu führt, dass das Pt in Lösung tritt, dort teilweise in Form von Nanopartikeln remetallisieren und die verbliebenen Hörsinneszellen angreifen kann.

WO 2019/206664 A1 offenbart einen Elektrodenkörper einer Elektrodenanordnung zur elektrischen Stimulation von Gewebe eines Lebewesens, insbesondere für Neurostimulation. Der Elektrodenkörper ist dazu eingerichtet, an einer Stelle zwischen dem Schädel und der Kopfschwarte eines Lebewesens angeordnet zu werden. Der Elektrodenkörper weist eine Stimulationsoberfläche auf, die dazu eingerichtet ist, in Kontakt mit dem Gewebe des Lebewesens gebracht zu werden, um eine elektrische Stimulation des Gewebes durch Wechselstrom- und/oder Gleichstrom-Pulse zu erzeugen. Der Elektrodenkörper hat eine Stimulationsoberfläche mit einem Flächeninhalt von wenigstens 50 mm$^2$ und zusätzlich eine die wirksame Stimulationsoberfläche vergrößernde Oberflächenbehandlung, eine Fixierungsstruktur für die Fixierung des Elektrodenkörpers am Gewebe eines Lebewesens, einen Leiteranschluss, über den der Elektrodenkörper durch Löten, Kleben, Schweißen oder eine anderweitige Verbindungstechnik mit einem elektrischen Leiter zur elektrischen Verbindung des Elektrodenkörpers mit einem elektrischen Gerät oder einem anderen Elektrodenkörper verbunden werden kann, der Elektrodenkörper ist bis auf die Stimulationsoberfläche von einem elektrisch isolierenden Trägermaterial vollständig umgeben und/oder der Elektrodenkörper hat über den Umfang verteilt angeordnete Ausnehmungen zur Fixierung des Elektrodenkörpers in einem Trägermaterial. Des weiteren sind Elektrodenanordnungen sowie ein Verfahren zur Herstellung einer Elektrodenanordnung angegeben.

EP 1 421 972 A2 offenbart eine Stimulationselektrode und deren Verwendung. Die Elektrodenoberfläche der Stimulationselektrode ist zumindest teilweise mit einer Beschichtung aus Titannitrid bedeckt, wobei das Titannitrid auf seiner der Elektrodenoberfläche abgewandten Seite eine höhere Oberfläche aufweist als der durch das Titannitrid bedeckte Bereich der Elektrodenoberfläche. Das Titannitrid ist erfindungsgemäß auf seiner der Elektrodenoberfläche abgewandten Seite mit mindestens einer Oxidationsschutzschicht bedeckt. Eine weitere Stimulationselektrode wird in WO 2016/130402 A1 offenbart.

Aufgabe der vorliegenden Erfindung

Ausgehend hiervon, besteht die Aufgabe der vorliegenden Erfindung darin, ein aktives implantierbares Medizinprodukt und ein Verfahren zu dessen Herstellung bereitzustellen, welche die aufgeführten Nachteile und Einschränkungen des Standes der Technik zumindest teilweise überwinden.

Insbesondere sollen in dem aktiven implantierbaren Medizinprodukt zur Stimulation eines Teils des Körpers, insbesondere von Nerven oder Muskeln, verwendete Elektroden eine deutlich verringerte Korrosion aufweisen und gleichzeitig Anforderungen an das aktive implantierbare Medizinprodukt an Biokompatibilität und eine hohe Ankopplung, bevorzugt eine hohe kapazitive Ankopplung, an die Gewebeumgebung erfüllen.

Offenbarung der Erfindung

Diese Aufgabe wird durch ein aktives implantierbares Medizinprodukt und ein Verfahren zu dessen Herstellung gemäß den Merkmalen der unabhängigen Ansprüche gelöst. Vorteilhafte Weiterbildungen, welche einzeln oder in beliebiger Kombination realisierbar sind, sind in den abhängigen Ansprüchen dargestellt.

Im Folgenden werden die Begriffe "haben", "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben den durch diese Begriffe eingeführten Merkmalen, keine weiteren Merkmale vorhanden sind, oder auf Situationen, in welchen ein oder mehrere weitere Merkmale vorhanden sind. Beispielsweise kann sich der Ausdruck "A hat B", "A weist B auf", "A umfasst B" oder "A schließt B ein" sowohl auf die Situation beziehen, in welcher, abgesehen von B, kein weiteres Element in A vorhanden ist (d.h. auf eine Situation, in welcher A ausschließlich aus B besteht), als auch auf die Situation, in welcher, zusätzlich zu B, ein oder mehrere weitere Elemente in A vorhanden sind, beispielsweise Element C, Elemente C und D oder sogar weitere Elemente.

Weiterhin wird darauf hingewiesen, dass die Begriffe "mindestens ein" und "ein oder mehrere" sowie grammatikalische Abwandlungen dieser Begriffe, wenn diese in Zusammenhang mit einem oder mehreren Elementen oder Merkmalen verwendet werden und ausdrücken sollen, dass das Element oder Merkmal einfach oder mehrfach vorgesehen sein kann, in der Regel lediglich einmalig verwendet werden, beispielsweise bei der erstmaligen Einführung des Merkmals oder Elementes. Bei einer nachfolgenden erneuten Erwähnung des Merkmals oder Elementes wird der entsprechende Begriff "mindestens ein" oder "ein oder mehrere" in der Regel nicht mehr verwendet, ohne dass hierdurch die Möglichkeit eingeschränkt wird, dass das Merkmal oder Element einfach oder mehrfach vorgesehen sein kann.

Weiterhin werden im Folgenden die Begriffe "vorzugsweise", "insbesondere", "beispielsweise" oder ähnliche Begriffe in Verbindung mit optionalen Merkmalen verwendet, ohne dass alternative Ausführungsformen hierdurch beschränkt werden. So sind Merkmale, welche durch diese Begriffe eingeleitet werden, optionale Merkmale, und es ist nicht beabsichtigt, durch diese Merkmale den Schutzumfang der Ansprüche und insbesondere der unabhängigen Ansprüche einzuschränken. So kann die Erfindung, wie der Fachmann erkennen wird, auch unter Verwendung anderer Ausgestaltungen durchgeführt werden. In ähnlicher Weise werden Merkmale, welche durch "in einer Ausführungsform der Erfindung" oder durch "in einem Ausführungsbeispiel der Erfindung" eingeleitet werden, als optionale Merkmale verstanden, ohne dass hierdurch alternative Ausgestaltungen oder der Schutzumfang der unabhängigen Ansprüche eingeschränkt werden soll. Weiterhin sollen durch diese einleitenden Ausdrücke sämtliche Möglichkeiten unangetastet bleiben, die hierdurch eingeleiteten Merkmale mit anderen Merkmalen zu kombinieren, seien es optionale oder nicht-optionale Merkmale.

In einem ersten Aspekt betrifft die vorliegende Erfindung ein aktives implantierbares Medizinprodukt zur Behandlung eines menschlichen oder tierischen Körpers. In Bezug auf die Begriffe "Medizinprodukt", "aktives Medizinprodukt" und "implantierbar" wird hierbei auf die eingangs vorgestellten Definitionen verwiesen. Wie ebenfalls dort erwähnt, ist das aktive implantierbare Medizinprodukt insbesondere ausgewählt aus der Gruppe umfassend Cochlea-Implantate, Retina-Implantate, implantierbare Kardioverter-Defibrillatoren, Blasen-Implantate zur Überwindung von Inkontinenz oder Implantate zur Tiefenhirn-Stimulation. Weitere Arten von aktiven implantierbaren Medizinprodukten sind jedoch möglich.

Wie eingangs bereits erwähnt, zeichnen sich die genannten aktiven implantierbaren Medizinprodukte insbesondere dadurch aus, dass sie mindestens eine elektrisch leitfähige Elektrode, die auch als "Stimulationselektrode" bezeichnet wird, aufweisen, die zur Stimulation einer zumindest teilweise der Elektrode zugewandten, d.h. insbesondere einer zumindest teilweise an die Elektrode angrenzenden, Gewebeumgebung im Körper, bevorzugt dort befindlicher Nerven oder Muskeln, eingerichtet ist. Im Folgenden wird die vorliegende Erfindung - ohne Beschränkung der Allgemeinheit - weitgehend anhand der Stimulationselektrode in einem Cochlea-Implantat, die zur Beaufschlagung des Hörnervs und/oder der Ganglien der Hörbahn eingerichtet ist, beschrieben.

Das aktive implantierbare Medizinprodukt umfasst

-   mindestens eine elektrisch leitfähige Elektrode zur Stimulation einer zumindest teilweise einer Oberfläche der Elektrode zugewandten Gewebeumgebung im Körper, wobei die mindestens eine Elektrode zumindest ein Elektrodenmaterial aufweist;
-   mindestens eine Energiequelle zur Bereitstellung von Energie zur Stimulation der Gewebeumgebung; und
-   mindestens eine Anbindung der mindestens einen Elektrode an die mindestens eine Energiequelle,

wobei die mindestens eine Elektrode ferner an der der Gewebeumgebung zugewandten Oberfläche der mindestens einen Elektrode eine biokompatible, unter Stimulationsbedingungen chemisch stabile Schicht mindestens einer metallhaltigen Verbindung aufweist.

Das aktive implantierbare Medizinprodukt kann einteilig oder mehrteilig aufgebaut sein. Beispielsweise kann die mindestens eine Energiequelle zur einfacheren Austauschbarkeit außerhalb des Körpers angebracht sein, während die mindestens eine Elektrode im Körper möglichst angrenzend an die entsprechende Gewebeumgebung implantiert sein kann. Darüber hinaus kann das aktive implantierbare Medizinprodukt mindestens eine, je nach Art und Einsatzbereich des Medizinprodukts, ausgewählte weitere Komponente umfassen. Bevorzugte Beispiele für derartige Komponenten umfassen eine Steuereinheit zur Ansteuerung der mindestens einen Elektrode und/oder der mindestens einen Energiequelle, eine Auswerteeinheit zur Bestimmung mindestens eine Wertes in Bezug auf die mindestens eine Elektrode und/oder die mindestens eine Energiequelle, oder eine Kommunikationseinheit zum Versenden und/oder Empfangen von Informationen.

Das erfindungsgemäße aktive implantierbare Medizinprodukt umfasst mindestens eine elektrisch leitfähige Elektrode. Der Begriff der "Elektrode" betrifft hierbei mindestens ein elektrisch leitfähiges Material, das auch als "Elektrodenmaterial" bezeichnet wird, welches zur Bereitstellung einer elektrischen Spannung oder eines elektrischen Stroms zum Zweck einer Beaufschlagung einer Umgebung der Elektrode, insbesondere einer zumindest teilweise einer Oberfläche der Elektrode zugewandten Gewebeumgebung im Körper, in welchen die Elektrode implantiert ist, eingerichtet ist. Wie unten näher ausgeführt, kann hierbei die Beaufschlagung der Gewebeumgebung mit einer elektrischen Spannung, welche von der elektrisch leitfähigen Elektrode erzeugt wird, besonders bevorzugt sein. Der Begriff "elektrisch leitfähig" betrifft hierbei eine Eigenschaft eines Stoffes, einen elektrischen Strom zu leiten, wobei ein Stoff mit einer elektrischen Leitfähigkeit von mindestens $10^3$ S/m als "metallisch" und mit einer elektrischen Leitfähigkeit von höchstens $10^{-6}$ S/m als "isolierend" bezeichnet wird. Im Zusammenhang mit der vorliegenden Erfindung wird darauf hingewiesen, dass die elektrische Leitfähigkeit in erster Linie die Leitung für Elektronen im ausgewählten Stoff betrifft; grundsätzlich ist jedoch auch eine Leitung von Ionen durch den Stoff denkbar.

In einer besonders bevorzugten Ausgestaltung kann die Elektrode derart in Bezug auf eine weitere Elektrode, die insbesondere als "Gegenelektrode" bezeichnet wird, angeordnet und mit ihr in Wechselwirkung stehen, dass zumindest ein Teil der Gewebeumgebung zwischen der Elektrode und der Gegenelektrode eingebracht ist und die gewünschte Beaufschlagung erfährt. Weisen die beiden Elektroden eine unterschiedliche Polarität auf und ist, wie im menschlichen oder tierischen Körper, das Gewebe elektrisch isolierend, so kann eine Anordnung von Elektrode, isolierender Gewebeumgebung und Gegenelektrode als "Kondensator" betrachtet werden, in dem das Paar aus Elektrode und Gegenelektrode ein elektrisches Feld erzeugt, das zumindest das dazwischen befindliche Gewebe mit einer elektrischen Spannung beaufschlagt, ohne dass hierbei ein nennenswerter Strom durch das Gewebe fließt, wodurch sich eine hohe kapazitive Ankopplung der mindestens einen Elektrode an die Gewebeumgebung erzielen lässt.

Die mindestens eine Elektrode kann grundsätzlich jede beliebige Form annehmen, bevorzugt ist jedoch eine die Schicht der metallhaltigen Verbindung tragende Oberfläche der Elektrode ebenfalls in Form einer Schicht. Der Begriff der "Schicht" bedeutet, dass zwei Dimensionen der Oberfläche der Elektrode die Dimensionen in die dritte Richtung, die auch als "Schichtdicke" bezeichnet wird, um mindestens einen Faktor 5, bevorzugt einen Faktor 20, besonders bevorzugt einen Faktor 50, übersteigen. Insbesondere kann die Elektrode eine Schichtdicke von 1 $\mu$m, bevorzugt von 5 $\mu$m, besonders

bevorzugt von 10 μm, bis 250 μm, bevorzugt bis 100 μm, besonders bevorzugt bis 50 μm aufweisen. Insbesondere um eine möglichst gute Ankopplung an die Gewebeumgebung zu erzielen, kann die mindestens eine Elektrode in Form einer Ringelektrode vorliegen. Der Begriff der "Ringelektrode" bezeichnet hier einen Elektrodenkörper in Form eines Rings, wobei eine nach außen gerichtete Oberfläche des Rings die Schicht der metallhaltigen Verbindung trägt. Hierbei können zueinander benachbarte Elektroden als Elektrode und Gegenelektrode ausgestaltet sein; alternativ oder zusätzlich kann eine in einer Spitze eines die Ringelektroden tragenden Stabs eingebrachte flächenförmige Elektrode die Elektrode bzw. Gegenelektrode ausbilden. Weitere Arten der Ausgestaltung der mindestens einen Elektrode sind denkbar.

Die mindestens eine elektrisch leitfähige Elektrode dient erfindungsgemäß zur Stimulation der zumindest teilweise einer Oberfläche der Elektrode zugewandten Gewebeumgebung im Körper. Der Begriff "zugewandt" bedeutet hierbei, dass eine Oberfläche der Elektrode zumindest teilweise auf ein Gewebe oder ein Teil hiervon hin ausgerichtet ist, wobei eine Oberfläche der Elektrode oder ein Teil hiervon insbesondere an das Gewebe oder den Teil hiervon angrenzen kann. Der Begriff der "Stimulation" bezeichnet hierbei grundsätzlich eine Anregung von Gewebe im menschlichen oder tierischen Körper, insbesondere eines Nervs oder eines Muskels, mittels eines exogenen Reizes, der im vorliegenden Falle mittels der von dem aktiven implantierbaren Medizinprodukt umfassten mindestens einen elektrisch leitfähigen Elektrode ausgelöst wird. Je nach Art des Gewebes und der Weise der Anregung des Gewebes, kann hierbei eine physiologische Aktivität im Körper ausgelöst werden. Ist das Gewebe ein Herzmuskel, so kann in dem Muskelgewebe mittels eines durch die mindestens eine Elektrode erzeugten elektrischen Feldes ein Aktionspotential hervorgerufen werden, das eine gewünschte Bewegung des Herzmuskels bewirkt. Ist das Gewebe dagegen ein Hörnerv oder eine Hörbahn, so kann in dem Hörnerv oder den Ganglien der Hörbahn mittels des durch die mindestens eine Elektrode erzeugten elektrischen Feldes eine Gehörinformation hervorgerufen werden, die bei Weiterleitung in den entsprechenden Bereich des Gehirns eine Gehörempfindung bewirken kann. Weitere Beispiele sind möglich.

Zur Bereitstellung von Energie, die zur Stimulation des Gewebes verwendet werden kann, verfügt das aktive implantierbare Medizinprodukt mindestens eine Energiequelle sowie mindestens eine Anbindung der mindestens einen Elektrode an die mindestens eine Energiequelle. Der Begriff "Energiequelle" bezeichnet hierbei eine Vorrichtung, die dazu eingerichtet ist, einen Energievorrat von außen übertragen zu bekommen und intern langfristig zu speichern und diesen zur Stimulierung an die mindestens eine Elektrode abzugeben. Die Energiequelle kann bevorzugt ausgewählt sein aus der Gruppe umfassend eine primäre Batterie, eine sekundäre Batterie, ein Solarzellenmodul oder einen kapazitiven Speicher.

Der Begriff der "Anbindung" betrifft eine Einrichtung, die für den Transport von Energie aus der mindestens einen Energiequelle zu der mindestens einen Elektrode eingerichtet ist. Die Anbindung kann hierbei bevorzugt als elektrisch leitfähige Zuleitung vorliegen, insbesondere in Form eines biokompatiblen Metalldrahtes, der bevorzugt Gold oder Silber umfassen und vorzugsweise von einer inerten Substanz, insbesondere einem Silikon, umgeben sein kann, um einerseits einen Kontakt mit einer Körperflüssigkeit zu unterbinden und andererseits in einem Multielektrodenimplantat, das mehrere Zuleitungen umfassen kann, einen Kurzschluss bei einem gegenseitigem Berühren von Zuleitungen zu verhindern. Beispielsweise kann die mindestens eine Elektrode mittels Kontaktschweißen auf der der Gewebeumgebung abgewandten Rückseite der Elektrode an die mindestens eine Anbindung angebracht werden. Hieran anschließend kann die so mit der mindestens einen Anbindung versehene Elektrode in eine dafür vorgesehene Form eingelegt und mit dem inerten Material, beispielsweise dem Silikon, bedeckt werden. Damit kann die mindestens eine elektrische leitfähige Anbindung in vorteilhafter Weise derart verpackt werden, dass das sich unter dem inerten Material in der Elektrode befindende Metall vor einer Auflösung in der Körperflüssigkeit geschützt werden kann. Alternativ oder zusätzlich kann die Anbindung der mindestens einen Elektrode an die mindestens eine Energiequelle mittels induktiver und/oder optischer Kopplung erfolgen. Weitere Arten der Anbindung sind möglich.

Erfindungsgemäß weist die mindestens eine Elektrode an der der Gewebeumgebung zugewandten Oberfläche der mindestens einen Elektrode eine biokompatible, unter Stimulationsbedingungen chemisch stabile Schicht einer metallhaltigen Verbindung auf. In Anlehnung an die Norm ISO 10993-1:2018 bezeichnet der Begriff "biokompatibel" eine hohe Verweilbarkeit der mindestens einen Elektrode in dem Gewebe des Körpers, in dem die mindestens eine Elektrode eingebracht ist, insbesondere über einen Zeitraum von mindestens 6 Monaten, bevorzugt von mindestens 2 Jahren, besonders bevorzugt von mindestens 10 Jahren, ohne dass während des Zeitraum eine Zersetzung oder toxische Wirkung der mindestens einen Elektrode oder eine hierdurch hervorgerufene Veränderung von Zellen in der Gewebeumgebung auftritt. Weiterhin bezeichnet der Begriff "chemisch stabil unter Stimulationsbedingungen", dass die durch die bei Stimulationsbedingungen durchgeführte Stimulation der Gewebeumgebung mittels der mindestens einen Elektrode bewirkte Wechselwirkung des Implantats mit dem Körper gering ist, so dass keine Abstoßungsreaktion des Körpers auf die mindestens eine implantierte Elektrode auftritt. Der Begriff der "Stimulationsbedingungen" bezeichnet hierbei mindestens einen Wert, bevorzugt eine Mehrzahl von Werten, in Bezug auf die Stimulation der jeweiligen Gewebeumgebung, abhängig vom ausgewählten Gewebe und der Art der vorgesehenen Stimulation. Da die Stimulation der Gewebeumgebung derart mittels der mindestens einen Elektrode erfolgt, dass hierdurch ein elektrisches Feld in der Gewebeumgebung hervorgerufen wird, können die Stimulationsbedingungen bevorzugt Werte in Bezug auf eine Amplitude, Pulsdauer und Wiederholrate des elektrischen Feldes bei der Beaufschlagung der Gewebeumgebung umfassen. Im Falle eines Cochlea-Implantats können bei einer Anregungsfrequenz von 300 Hz bis 3 kHz die Pulsdauern von 0,3 ms bis 3 ms betragen. Hierbei können kurze Pulsdauern besonders bevorzugt sein, insbesondere um längere Pausen zwischen aufeinanderfolgenden Pulsen erzeugen zu können. Außerdem werden Pulse, die längere Pulsdauern aufweisen, kaum in die Cochlea übertragen. Für andere aktive implantierbare Medizinprodukte können andere Werter zutreffen. Die Heranziehung weiterer Parameter ist jedoch möglich. Außerdem kann bei einem Vorhandensein einer Elektrode und einer Gegenelektrode unterschiedlicher Polarität die Richtung des elektrischen Feldes bei der Beaufschlagung der Gewebeumgebung geändert werden.

In einer besonders bevorzugten Ausgestaltung ist die der Gewebeumgebung zugewandte Oberfläche der mindestens einen Elektrode korrosionsbeständig. Hierbei betrifft der Begriff "korrosionsbeständig" eine Eigenschaft eines Materials, gemäß der eine Menge an Material, das während des Zeitraums in die Gewebeumgebung eingetragen werden kann, keinen in Bezug auf das Material vorgegebenen Grenzwert überschreitet.

Erfindungsgemäß wird daher vorgeschlagen, die der Gewebeumgebung zugewandte Oberfläche der mindestens einen Elektrode mit mindestens einer biokompatiblen, unter den Stimulationsbedingungen chemisch stabilen Schicht mindestens einer metallhaltigen Verbindung auszustatten. Der Begriff der "metallhaltigen Verbindung" bezeichnet hierbei mindestens ein Oxid, ein Hydroxid, ein Nitrid, ein Karbid, ein Oxykarbid, oder eine Kombination hiervon mindestens eines Metalls. Beispielsweise kann die Schicht ein einzelnes Oxid oder Nitrid eines einzelnen Metalls umfassen. Alternativ können mindestens zwei verschiedene Oxide eines Metalls vorhanden sein, sofern das Metall mehr als ein Oxid ausbilden kann. Alternativ oder zusätzlich kann die Schicht mindestens zwei unterschiedliche Oxide, Hydroxide, Nitride, Karbide oder Oxykarbide von mindestens zwei verschiedenen Metallen aufweisen. Wie unten näher erläutert, kann es vorteilhaft sein, wenn die Schicht mindestens zwei voneinander verschiedene metallhaltige Verbindungen umfasst. Unabhängig von der Art ihrer Zusammensetzung, ist die Schicht der metallhaltigen Verbindung zumindest auf der Oberfläche der mindestens einen Elektrode ausgebildet, so dass die Gewebeumgebung der Elektrode nur mit der Schicht der mindestens einen metallhaltigen Verbindung in Kontakt treten kann.

In einer besonders bevorzugten Ausgestaltung kann die Elektrode daher ein elektrisch leitfähiges Substrat aufweisen, wobei zumindest auf dessen der Gewebeumgebung zugewandten Oberfläche die Schicht der metallhaltigen Verbindung, unmittelbar oder auf mindestens einer dazwischenliegenden Haftvermittlerschicht, in Form einer Lage auf dem Substrat aufgebacht ist. In einer besonderen Ausgestaltung kann auf einer bereits vorhandenen ersten Schicht einer metallhaltigen Verbindung, unmittelbar oder auf mindestens einer dazwischenliegenden weiteren Haftvermittlerschicht, eine weitere Schicht einer metallhaltigen Verbindung aufgebracht sein, deren Zusammensetzung sich vorzugsweise von der ersten Schicht unterscheiden kann. Auf diese Weise können verschiedene vorteilhafte Eigenschaften von Schichten unterschiedlicher Zusammensetzung in einer Elektrode Verwendung finden.

In einer besonders bevorzugten Ausgestaltung kann das elektrisch leitfähige Substrat ein Elektrodenmaterial, das mindestens ein ausgewähltes Metall umfasst, und die mindestens eine Schicht einer metallhaltigen Verbindung, d.h. mindestens ein Oxid, ein Hydroxid, ein Nitrid, ein Karbid, ein Oxykarbid oder eine Kombination hiervon, des mindestens einen ausgewählten Metalls aufweisen. Diese besonders bevorzugte Ausgestaltung ermöglicht insbesondere, wie unten näher ausgeführt, eine einfache Herstellung der Elektrode mittels Bereitstellen eines Substrats, welches das ausgewählte Metall umfasst, und anschließender Behandlung der Oberfläche des Substrats, insbesondere mittels Anodisierung, zur Ausbildung der Schicht der metallhaltigen Verbindung aus dem ausgewählten Metall. Beispielsweise kann eine Elektrode, welche ein ausgewähltes Metall umfasst, bereitgestellt werden und auf deren Oberfläche durch Anodisierung eine Schicht der metallhaltigen Verbindung erzeugt werden. Alternativ oder zusätzlich kann die mindestens eine Metallschicht oder die Schicht der metallhaltigen Verbindung, insbesondere mittels eines unten näher beschriebenen additiven Verfahrens, auf das Substrat, auf eine Haftvermittlerschicht oder auf eine bereits vorhandene Metallschicht oder Schicht der metallhaltigen Verbindung aufgebracht werden. Auf diese Weise aufgebrachte Metallschichten können dann anschließend einer Behandlung, insbesondere einer Oxidation, unterzogen werden, um die gewünschte Schicht der metallhaltigen Verbindung, insbesondere eine Metalloxidschicht, zu erzeugen. Weitere Verfahren zur Ausbildung der Schicht der metallhaltigen Verbindung sind jedoch denkbar.

Insbesondere in der besonders bevorzugten Ausgestaltung, in der die Schicht mindestens eine metallhaltige Verbindung, ausgewählt aus mindestens einem Oxid, einem Hydroxid, einem Nitrid, einem Karbid, einem Oxykarbid oder einer Kombination hiervon, des mindestens einen ausgewählten Metalls aufweist, kann das Elektrodenmaterial vorzugsweise ausgewählt sein aus der Gruppe enthaltend Titan, Zirkonium, Hafnium, Niob, Tantal, Iridium und Ruthenium, wobei die Schicht mindestens eine metallhaltigen Verbindung, d.h. mindestens ein Metalloxid, ein Metallhydroxid, ein Metallnitrid, ein Metallkarbid, ein Metalloxykarbid oder eine Kombination hiervon, eines der Elektrodenmaterialien aufweist. Insbesondere die Metalloxide und die Metallnitride dieser Elektrodenmaterialien sind dafür bekannt, dass die unter Stimulationsbedingungen erzeugten Metallionen und Metalle in reduzierter Form, auch in Form von Nanopartikeln, biokompatible Eigenschaften aufweisen. Hierzu gehören die der "Platingruppe" zugeordneten Metalle Rhodium und Iridium sowie die auch als "Ventilmetalle" bezeichneten Übergangsmetalle Titan, Zirkonium, Hafnium, Niob und Tantal. Die ausgewählten Metalle sind vorteilhaft, da sie unter Stimulationsbedingungen jeweils eine chemisch stabile Metalloxidschicht ausbilden. Mit Ausnahme von Rhodium und Iridium, die elektrisch gut leitende Oxide ausbilden können, sind die übrigen zu dieser Gruppe gehörenden Elektrodenmaterialien elektrisch isolierend oder halbleitend und ermöglichen es daher, während der Stimulation ein Fließen von elektrischen Strömen durch die Metalldeckschicht zu vermeiden, dass sich jedoch eine hohe kapazitive Ankopplung an die Gewebeumgebung erzielen lässt. In einer besonderen Ausgestaltung der vorliegenden Erfindung kann die Elektrode ein Substrat aus einem der genannten Elektrodenmaterialien, beispielsweise Rhodium, Tantal oder Niob, umfassen, wobei zumindest auf dessen der Gewebeumgebung zugewandten Oberfläche, insbesondere mittels Anodisierung eine Schicht aus zumindest einem Oxid oder Nitrid des mindestens einen Elektrodenmaterials, hier Rutheniumoxid, Tantaloxid, Tantalnitrid oder Nioboxid, ausgebildet ist. Weitere Ausgestaltungen der mindestens einen Elektrode sind denkbar.

Insbesondere in derjenigen Ausgestaltung, in welcher die Schicht der metallhaltigen Verbindung additiv auf das Substrat aufgebracht ist, kann das Substrat der mindestens einen Elektrode mindestens ein Substratmaterial umfassen, wobei das Substratmaterial vorzugsweise ausgewählt ist aus der Gruppe enthaltend Gold, rostfreier Edelstahl, Titan, Zirkonium, Hafnium, Niob, Tantal, Ruthenium, Rhodium und Iridium. In einer besonderen Ausgestaltung der vorliegenden Erfindung kann die Elektrode ein Substrat aus einem Substratmaterial, beispielsweise Titan, umfassen, wobei das Substrat mit einer, zwei, drei, vier oder mehreren Schichten von metallhaltigen Verbindungen überzogen ist. Das für das Substrat gewählte Substratmaterial Titan oder rostfreier Edelstahl kann hierbei insbesondere eine mechanische Stabilität des Substrats erhöhen. Um eine mechanische Bearbeitbarkeit des Substrats zu erhöhen, kann das Substratmaterial eine Legierung aus einem ersten Substratmaterial, insbesondere Titan, mit einem zweiten Substratmaterial, insbesondere Niob oder Tantal, umfassen. Vorzugsweise kann hierdurch eine Phase, zum Beispiel die für diesen Zweck besonders geeignete kubische Phase des Titans, die auch kristallografische β-Phase bezeichnet wird, stabilisiert werden. Weiter vorzugsweise können Titan-Niob oder Titan-Tantal eingesetzt werden, in denen die kubische Phase des Titans stabilisiert ist, wodurch das sonst besonders schwer zu bearbeitende Material vergleichsweise leicht zu dünnen Blechen auswalzbar ist. Weiter beispielsweise können eine erste Metalloxidschicht, zum Beispiel Hafniumoxid, auf die Oberfläche des Substrats oder auf eine dazwischenliegende Haftvermittlerschicht und eine zweite Metalloxidschicht, beispielsweise Tantaloxid oder Nioboxid, auf die erste Metalloxidschicht oder auf eine ggf. weitere dazwischenliegende Haftvermittlerschicht aufgebracht sein. In dieser Ausgestaltung kann die erste Schicht der als die biokompatible Schicht dienen, während das Hafniumoxid der zweiten Schicht eine Flächenkapazität der Elektrode erhöhen kann.

Der Begriff der "Flächenkapazität" bezeichnet gemäß Gleichung (1) eine flächenbezogene Kapazität

$$C/A = \varepsilon_0\varepsilon_r/d \qquad (1)$$

der Elektrode, wobei C die Kapazität, A die Oberfläche der Elektrode, d die Schichtdicke der Elektrode, $\varepsilon_r$ eine relative Permittivität des Elektrodenmaterials und $\varepsilon_0$ die elektrische Feldkonstante angeben. Eine hohe Flächenkapazität bedeutet daher bei gleichen Dimensionen der Elektrode eine hohe relative Permittivität des Elektrodenmaterials. Beispielsweise kann eine Beimengung einer geringen Menge, insbesondere höchstens 25 %, bevorzugt höchstens 10 %, besonders bevorzugt höchstens 5 %, eines zweiten Metalls, insbesondere eines Edelmetalls, zum Beispiel Iridium oder Gold, zu einem ersten Metall, zum Beispiel Tantal oder Niob, dazu verwendet werden, die relative Permittivität des Elektrodenmaterials bei gleichzeitig erhaltener Korrosionsstabilität zu erhöhen. Weitere Ausgestaltungen der mindestens einen Elektrode sind denkbar.

Weiterhin können Oxide des Zirkonium und Hafnium bevorzugt sein, da hier chemische Oberflächenreaktionen im Wesentlichen über Sauerstoffreaktionen ablaufen, insbesondere durch eine hohe Mobilität von Sauerstoff-Ionen. Die Wahrscheinlichkeit, dass Zirkonium oder Hafnium in metallischer Form oder als metallischer Komplex aus dem freigesetzt werden, ist folglich geringer als bei den anderen Metalloxiden, zum Beispiel Tantaloxid.

Im direkten Vergleich unter gleichen Stimulationsbedingungen ist die Menge an Metall, die durch die elektrische Stimulation herausgelöst werden kann, bei Tantal am geringsten, bei Niob etwa um einen Faktor 10 höher im Vergleich zu Tantal, während sie bei Titan etwa um einen Faktor 100 höher ist im Vergleich zu Tantal.

Insbesondere kann die Schicht der mindestens einen metallhaltigen Verbindung eine Schichtdicke von 0,1 nm, bevorzugt von 0,2 nm, besonders bevorzugt von 0,5 nm, bis 2,5 nm, bevorzugt bis 2 nm, besonders bevorzugt bis 1,5 nm, aufweisen, wobei die Schichtdicke der Schicht der mindestens einen metallhaltigen Verbindung bevorzugt höchstens 10 %, bevorzugt höchstens 2 %, besonders bevorzugt höchstens 0,5 %, der Schichtdicke der Elektrode aufweisen kann. Die Schichtdicke des Substrats einschließlich einer optionalen Haftvermittlerschicht zwischen dem Substrat und der Schicht der mindestens einen metallhaltigen Verbindung und/oder zwischen zwei benachbart angeordneten Schichten der mindestens einen metallhaltigen Verbindung kann hierbei insbesondere aus einer Differenz zwischen der Schichtdicke der Elektrode und der Schichtdicke der Schicht der mindestens einen metallhaltigen Verbindung ermittelt werden. Für den Begriff der "Schichtdicke" wird auf die obige Definition verwiesen.

In einer bevorzugten Ausgestaltung der vorliegenden Erfindung ist es besonders vorteilhaft, wenn das für die Schicht der metallhaltigen Verbindung ausgewählte Material über eine hohe relative Permittivität $\varepsilon_r$ verfügt. Wie unten näher erläutert, weisen so genannten "high-k-Dielektrika" eine hohe relative Permittivität auf, welche nach üblicher Definition die relative Permittivität $\varepsilon_r \approx 3,9$ von Siliziumdioxid übersteigt. Zu den high-k-Dielektrika gehören insbesondere Oxide des Titan, Zirkonium, Hafnium, Tantal und Niob, i.e. $TiO_{2-x}$, $ZrO_{2-x}$, $HfO_{2-x}$, $Ta_2O_{5-x}$ und $Nb_2O_5$ mit $0 \leq x < 0,1$, wobei die Oxide des Zirkonium, Hafnium, Tantal und Niob aus den oben genannten Gründen besonders bevorzugt sind.

In einer bevorzugten Ausgestaltung der vorliegenden Erfindung ist es besonders vorteilhaft, wenn die für die Schicht ausgewählte metallhaltigen Verbindung eine geringe Delamination in Bezug auf das Substrat, auf welchem es aufgebracht wird, aufweist. Der Begriff der "Delamination" bezeichnet eine Ablösung, welche üblicherweise davon beeinflusst wird, in welchem Maße sich die Schicht aus der metallhaltigen Verbindung unter intrinsischen Stress bildet. Je nach Ausmaß des intrinsischen Stresses können hierbei Ablösungskräfte auftreten. Neben spezifischen Eigenschaften der Grenzfläche, insbesondere einem Verlauf und einer Morphologie der Einlagerung der Verbindung in ein Metallgitter oder umgekehrt einer von Anionen und Kationen in dem Metallgitter, spiegelt sich der Stress in einem Volumenverhältnis von Metall zu gebildeter Verbindung wider. Hierfür existiert ein als "Pilling-Bedworth-Ratio", abgekürzt "PBR", bezeichnetes Maß, welches im Idealfall bei 1,0 liegen sollte. In bevorzugter Weise eignen sich hierfür Oxide des Titan, Zirkonium, Hafnium und weniger des Tantal oder Niob, i.e. $TiO_{2-x}$ (PBR = 1,73), $ZrO_{2-x}$ (PBR = 1,56), $HfO_{2-x}$ (PBR = 1,62), $Ta_2O_{5-x}$ (PBR = 2,47) und $Nb_2O_{5-x}$ (PBR = 2,69) mit $0 \leq x < 0,1$, die den jeweils angegebenen PBR-Wert für aufweisen, wobei auch hier aus den oben genannten Gründen Oxide des Zirkonium, Hafnium und Tantal besonders bevorzugt sind.

Zusammengefasst können die folgenden Gesichtspunkte vorzugsweise bei Auswahl der mindestens einen metallhaltigen Verbindung für die Schicht berücksichtigt werden:

- Biokompatibilität der metallhaltigen Verbindung und vorzugsweise auch des Materials der darunterliegenden Elektrode;
- möglichst geringe Auflösung von Metallionen aus dem für die mindestens Elektrode verwendeten Materials unter Stimulationsbedingungen;
- Art der Ladungsträger in dem Material, wobei anionenleitende Materialien besonders bevorzugt sind;
- hohe relative Permittivität der metallhaltigen Verbindung, um eine Optimierung von schützender Schicht und maximalem Ladungstransfer zu möglichen;
- mechanische Stabilität der aus der metallhaltigen Verbindung gebildeten Schicht als Schutz vor Delamination.

Um ein möglichst geeignetes Material für die Schicht zu erhalten, kann das Material somit vorzugsweise auch mehr als eine metallhaltige Verbindung umfassen.

Unabhängig von der Schichtdicke und der Art der der Schicht der metallhaltigen Verbindung kann die damit versehene und der Gewebeumgebung zugewandte Oberfläche der Elektrode grundsätzlich eine beliebige Ausgestaltung aufweisen. In einer bevorzugten Ausgestaltung kann die Oberfläche der Schicht der metallhaltigen Verbindung in Form einer planaren Ebene vorliegen. Diese Art der Ausgestaltung kann in vorteilhafter Weise mittels eines einfachen Verfahrens herstellbar sein.

In einer besonderen Ausgestaltung kann die Oberfläche der Schicht der metallhaltigen Verbindung jedoch eine von einer planaren Ebene abweichende Oberflächentopographie aufweisen. Hierzu kann die Oberfläche der Schicht der metallhaltigen Verbindung mit einer Rauheit oder alternativ mit einer periodischen Struktur, insbesondere ausgewählt aus einer Wellenstruktur oder einem Profil, zum Beispiel einem Dreieck- oder einem Rechteckprofil, versehen werden, wobei die periodische Struktur über eine charakteristische Länge, die in Beziehung zu einer Periode der Struktur steht, zum Beispiel einen Abstand zwischen zwei Maxima oder Minima der Wellenstruktur, oder eine Länge eines Basisvektors oder einer Einheitszelle in dem Profil, verfügen kann. Die Oberflächentopographie kann mittels eines additiven Verfahrens oder, vorzugsweise, mittels eines subtraktiven Verfahrens in die Oberfläche der Schicht der metallhaltigen Verbindung eingebracht werden. Diese besondere Ausgestaltung kann insbesondere den Vorteil aufweisen, dass die damit ausgestattete Elektrode über eine höhere effektive Oberfläche und damit eine höhere Flächenkapazität verfügen kann. Auf diese Weise kann eine Verringerung der Kapazität in Folge der erfindungsgemäß für die Elektroden verwendeten metallhaltigen Verbindung im Vergleich zu dem bisher verwendeten Platinoxid zumindest teilweise kompensiert werden.

Diese Art der Ausgestaltung kann besonders vorteilhaft für ein Cochlea-Implantat sein, wenn die charakteristische Länge einerseits die Schichtdicke einer Helmholtz-Schicht in der Grenzschicht zwischen Schicht der metallhaltigen Verbindung und der Perilymphe im Innenohr deutlich übersteigt, und andererseits deutlich geringer ist als eine Skalenlänge eines Abfalls des elektrischen Feldes in der Anordnung aus der mindestens einen Elektrode und der Gewebeumgebung während der Stimulation. Der Begriff der "Helmholtz-Schicht" bezeichnet hierbei einen Bereich in der Perilymphe, welcher unmittelbar an die Elektrode angrenzt und dadurch eine Überschussladung mit umgekehrtem Vorzeichen in Bezug auf die Ladung der Elektrode trägt. Weiterhin gibt der Begriff der "Skalenlänge" hierbei einen Wert für eine Länge, über die das elektrische Feld auf einen vorgegebenen Wert abfällt, beispielsweise auf 1/e, wobei e die Eulersche Zahl ist, auf 10 % oder auf 1 %. Andere Werte sind denkbar.

Darüber hinaus kann das aktive implantierbare Medizinprodukt weitere Bestandteile umfassen, die spezifisch in Bezug auf die vorgesehene Verwendung sein können. Bevorzugt kann eine Steuerungseinheit vorgesehen sein, die dazu eingerichtet ist, die Stimulation der jeweiligen Gewebeumgebung zu steuern, insbesondere zur Bereitstellung der Stimulationsbedingungen, insbesondere in Bezug auf Amplitude, Pulsdauer und/oder Wiederholrate des elektrischen Feldes bei der Beaufschlagung der Gewebeumgebung. Weiterhin kann eine Auswerteeinheit vorgesehen sein, die dazu eingerichtet ist, Messwerte, die in Zusammenhang mit der Stimulation der jeweiligen Gewebeumgebung stehen, aufzunehmen und hieraus Werte zu bestimmen, die an die Steuerungseinheit und/oder an eine externe Einheit, zum Beispiel zur Information für den Patienten oder einen Behandler des Patienten, ausgegeben werden können. Weitere Bestandteile sind möglich.

In einem weiteren Aspekt betrifft die vorliegende Erfindung ein Verfahren zur Herstellung eines aktiven implantierbaren Medizinprodukts zur Behandlung eines menschlichen oder tierischen Körpers, insbesondere zur Herstellung des in diesem Dokument beschriebenen aktiven implantierbaren Medizinprodukts. Das Verfahren umfasst hierbei zumindest die folgenden Verfahrensschritte:

a) Bereitstellen mindestens einer elektrisch leitfähigen Elektrode;
b) Behandeln der Oberfläche der Elektrode, wobei die biokompatible, unter Stimulationsbedingungen chemisch stabile Schicht mindestens einer metallhaltigen Verbindung ausgebildet wird; und
c) Herstellen mindestens einer Anbindung der mindestens einen Elektrode an die mindestens eine Energiequelle zur Bereitstellung von Energie zur Stimulation einer zumindest teilweise der Schicht der mindestens einen metallhaltigen Verbindung zugewandten Gewebeumgebung im Körper.

Hierbei können die Verfahrensschritte a) bis c) nacheinander in der angegebenen Reihenfolge a), b) und c) oder in einer hiervon abweichenden Reihenfolge, insbesondere in der Reihenfolge a), c) und b), durchgeführt werden. Dadurch kann das Herstellen der mindestens einen Anbindung gemäß Schritt c), die in Form einer elektrisch leitfähigen Zuleitung ausgestaltet sein kann, insbesondere vor oder nach dem Behandeln der Oberfläche der Elektrode gemäß Schritt b) erfolgen.

Gemäß Schritt a) wird mindestens eine elektrisch leitfähige Elektrode bereitgestellt. Hierbei kann die Elektrode, wie oben oder unten beschrieben, vorzugsweise ein Elektrodenmaterial umfassen, das aus der Gruppe enthaltend Titan, Zirkonium, Hafnium, Niob, Tantal, Iridium und Rhodium ausgewählt wird. In einer alternativen Ausgestaltung kann die mindestens eine Elektrode mindestens ein elektrisch leitfähiges Substratmaterial aufweisen, das vorzugsweise aus der Gruppe enthaltend Gold, Stahl, Titan, Zirkonium, Hafnium, Niob, Tantal, Ruthenium, Rhodium und Iridium ausgewählt wird. Für weitere Einzelheiten wird auf die oben und die untenstehende Beschreibung verwiesen.

Gemäß Schritt b) erfolgt ein Behandeln der Oberfläche der Elektrode, wobei die biokompatible, unter Stimulationsbedingungen chemisch stabile Schicht der metallhaltigen Verbindung erzeugt wird. In einer besonders bevorzugten Ausgestaltung kann hierzu aus einem der genannten Elektrodenmaterialien, zum Beispiel Rhodium, Tantal oder Niob, zumindest auf der Oberfläche, die der Gewebeumgebung zugewandt angeordnet werden soll, insbesondere mittels Anodisierung, eine Schicht einer metallhaltigen Verbindung, d.h. mindestens eines Oxid, eines Hydroxid, eines Nitrid, eines Karbid, eines Oxykarbids oder einer Kombination hiervon, des mindestens einen Elektrodenmaterials, insbesondere Rhodiumoxid, Tantaloxid, Tantalnitrid oder Nioboxid, ausgebildet werden. Der Begriff "Anodisierung" bezeichnet hierbei ein elektrolytisches Verfahren, welches dazu eingesetzt wird, eine Schicht der metallhaltigen Verbindung auf der Oberfläche eines Metalls herzustellen oder die Schichtdicke einer Schicht der metallhaltigen Verbindung, die bereits auf der Oberfläche des Metalls vorhanden ist, zu erhöhen, wobei unter Einsatz eines auch als "Anodisierungsmedium" bezeichneten Elektrolyten eine oberflächennahe Schicht des Metalls in die mindestens eine metallhaltige Verbindung des betreffenden Metalls umgewandelt, d.h. oxidiert, wird. Die Anodisierung weist insbesondere in den Randbereichen den Vorteil auf, dass mittels einer Feldüberhöhung an Graten und scharfen Ecken die Ausbildung der metallhaltigen Verbindung verstärkt ausfallen kann.

In einer besonders vorteilhaften Ausgestaltung kann die Anodisierung in einem Elektrolyten erfolgen, der mindestens einen Puffer umfasst, wobei der Puffer ausgewählt ist aus einem Acetatpuffer oder einem Citratpuffer. Acetatpuffer, Phosphatpuffer und ihre Spuren sind biokompatibel und ermöglichen eine Durchführung der Anodisierung in neutralen Lösungen, ohne dass ein Einsatz von Fluorid-Ionen oder Chlorid-Ionen, die als Komplexbildner wirken und in üblichen Anodisierungsmedien wie $H_2SO_4$ auftreten, oder von auch Borsäure, die wegen möglicher Borspuren in der erzeugten metallhaltigen Verbindung nachteilig ist, erforderlich wäre.

Hierbei kann die Anodisierung eines Teils des Elektrodenmaterials bei einer Anodisierungsspannung erfolgen, welche eine gemäß den Stimulationsbedingungen zum Beaufschlagen der der Schicht der metallhaltigen Verbindung zugewandten Gewebeumgebung im Körper festgelegte elektrische Spannung übersteigt. Zur Durchführung der Anodisierung kann es besonders vorteilhaft sein, wenn die metallhaltige Verbindung eine ausreichende elektrische Festigkeit, insbesondere eine hohe dielektrische Durchbruchsfestigkeit, unter Stimulationsbedingungen aufweist. Zu diesem Zweck, kann als Anodisierungsspannung vorzugsweise ein Wert gewählt werden, welcher mindestens dem 1,25-fachen, bevorzugt mindestens dem 1,5-fachen, besonders bevorzugt mindestens dem 2-fachen der bei der Anwendung des aktiven implantierbaren Medizinprodukts zu erwartenden maximalen Stimulationsspannung liegt.

Vorzugsweise kann hierbei die Anodisierung des Elektrodenmaterials bei einer Rampe der Anodisierung von 10 mV/s, bevorzugt von 20 mV/s, besonders bevorzugt von 25 mV/s, bis 100 mV/s, bevorzugt bis 80 mV/s, besonders bevorzugt bis 50 mV/s, erfolgen, wobei eine Haltezeit von 100 s, bevorzugt von 200 s, besonders bevorzugt von 500 s, bis 10000 s, bevorzugt bis 5000 s, besonders bevorzugt bis 2000 s, bei einem Maximum der Anodisierungsspannung eingehalten werden kann, wobei das Elektrodenmaterial mit Spannungspulsen von 10 V, bevorzugt von 20 V, besonders bevorzugt von 25 V, bis 100 V, bevorzugt bis 80 V, besonders bevorzugt bis 75 V, oberhalb der Anodisierungsspannung während mindestens einer Zeitdauer von 0,1 ms, bevorzugt von 0,5 ms, besonders bevorzugt von 1 ms, bis 10 ms, bevorzugt bis 8 ms, besonders bevorzugt bis 5 ms, beaufschlagt wird. Ein derartig hergestelltes Metalloxid kann insbesondere ein hochverdichtetes Metalloxid mit sehr geringer Fehlstellenzahl sein, dessen Durchbruchfeldstärke höher liegen kann als die Bildungsfeldstärke, die üblicherweise als der maximal erreichbare Wert für die Feldstärke betrachtet wird. Hieraus ergibt sich, dass die Durchbruchfeldstärke höher sein kann als ein reziproker Wert eines unten näher erläuterten Schichtbildungsfaktors.

Alternativ oder zusätzlich kann die mindestens eine Schicht der metallhaltigen Verbindung insbesondere mittels eines additiven Verfahrens auf die Oberfläche das Substrat, auf eine Haftvermittlerschicht oder auf eine bereits vorhandene Schicht der metallhaltigen Verbindung aufgebracht werden. Der Begriff des "additiven Verfahrens" bezeichnet ein Herstellungsverfahren, bei welchem ein zusätzliches Material auf eine bestehende Oberfläche aufgebracht wird. Für Schritt b) des vorliegenden Verfahrens eignet sich hierbei bevorzugt ein Verfahren ausgewählt aus Atomlagen-Abscheidung (engl. *atomic layer deposition,* ALD), physikalischer Gasphasenabscheidung (engl. *physical vapor deposition,* PVD) oder chemischer Gasphasenabscheidung (engl. *chemical vapor,* CVD). Ein Einsatz weiterer additiver Verfahren ist denkbar, insbesondere von Vakuumverdampfung, Sputtern, Spray-Pyrolyse, Elektroabscheidung oder chemischer Badabscheidung.

Gemäß Schritt c) erfolgt ein Herstellen mindestens einer Anbindung der mindestens einen Elektrode an die mindestens eine Energiequelle zur Bereitstellung von Energie zur Stimulation einer zumindest teilweise der Schicht der metallhaltigen Verbindung zugewandten Gewebeumgebung im Körper. Hierbei kann die mindestens eine Anbindung eine biokompatible metallische Zuleitung umfassen, vorzugsweise ausgewählt aus Gold oder Silber, die vorzugsweise von einer inerten Substanz, insbesondere einem Silikon, umgeben sein kann. Darüber hinaus können weitere Zuleitungen zu weiteren Bestandteilen des aktiven Medizinprodukts, insbesondere zu einer Steuerungseinheit und/oder zu einer Auswerteeinheit, hergestellt werden. Alternativ oder zusätzlich kann jedoch auch eine induktive und/oder optische Kopplung eingesetzt werden.

Für weitere Einzelheiten zum vorliegenden Verfahren und zu hierin erwähnten Merkmalen wird auf die obige Beschreibung des aktiven implantierbaren Medizinprodukts sowie auf die Ausführungsbeispiele verwiesen.

In einem weiteren Aspekt betrifft die vorliegende Erfindung ein Verfahren zur Behandlung des menschlichen oder tierischen Körpers, umfassend die Verfahrensschritte:

- Bereitstellen eines aktiven implantierbaren Medizinprodukts gemäß Anspruch 1, wobei das aktive implantierbare Medizinprodukt mindestens eine Elektrode umfasst;
- Implantieren mindestens eines Teils des aktiven implantierbaren Medizinprodukts derart in den Körper, dass eine Oberfläche der mindestens einen Elektrode einer durch die mindestens eine Elektrode zu stimulierenden Gewebeumgebung im Körper zugewandt ist;
- Behandeln des Körpers mittels Stimulation der Gewebeumgebung im Körper, die zumindest teilweise der Oberfläche der mindestens einen Elektrode zugewandt ist.

Für weitere Einzelheiten in Bezug auf die hierin beschriebenen Verfahren wird auf die Beschreibung des aktiven implantierbaren Medizinprodukts verwiesen.

In einem weiteren Aspekt betrifft die vorliegende Erfindung eine Verwendung des hierin vorgestellten aktiven implantierbaren Medizinprodukts insbesondere in einem Cochlea-Implantat, Retina-Implantat, implantierbaren Kardioverter-Defibrillator, Blasen-Implantat zur Überwindung von Inkontinenz oder Implantat zur Tiefenhirn-Stimulation. Weitere Verwendungen sind jedoch möglich. Beispielsweise kann das aktive implantierbare Medizinprodukt als Cochlea-Implantat dazu dienen, in dem Hörnerv und/oder den Ganglien der Hörbahn eine Gehörinformation hervorzurufen, die bei Weiterleitung in den entsprechenden Bereich des Gehirns eine Gehörempfindung bewirken kann. Alternativ kann das aktive implantierbare Medizinprodukt als Herzschrittmacher zur Stimulation eines Herzmuskels eingesetzt werden, um in dem Muskelgewebe ein Aktionspotential hervorzurufen, das eine gewünschte Bewegung des Herzmuskels bewirkt. Weitere Beispiele sind möglich.

Vorteile der Erfindung

Das hierin vorgeschlagene aktive implantierbare Medizinprodukt und das zugehörige Herstellungsverfahren weisen, im Vergleich zu aus dem Stand der Technik bekannten aktiven implantierbaren Medizinprodukten und zugehörigen Herstellungsverfahren eine Reihe von Vorteilen auf. Sie ermöglichen insbesondere, dass die bei der Stimulation eines Teils des Körpers wie Nerven oder Muskeln verwendeten Elektroden eine deutlich verringerte Korrosion aufweisen und gleichzeitig Anforderungen an das aktive implantierbare Medizinprodukt an Biokompatibilität und eine hohe Ankopplung, insbesondere eine hohe kapazitive Ankopplung, an die Gewebeumgebung, erfüllen.

Kurze Beschreibung der Figuren

Weitere Einzelheiten und Merkmale der vorliegenden Erfindung ergeben sich aus der nachfolgenden Beschreibung von bevorzugten Ausführungsbeispielen, insbesondere in Verbindung mit den abhängigen Ansprüchen. Hierbei können die jeweiligen Merkmale für sich alleine oder zu mehreren in Kombination miteinander verwirklicht sein. Die Erfindung ist jedoch nicht auf die Ausführungsbeispiele beschränkt. Die Ausführungsbeispiele sind schematisch in den nachfolgenden Figuren dargestellt. Hierbei bezeichnen gleiche Bezugsziffern in den Figuren gleiche oder funktionsgleiche Elemente bzw. hinsichtlich ihrer Funktionen einander entsprechende Elemente.

Im Einzelnen zeigen:

Figur 1    eine schematische Darstellung einer bevorzugten Ausführung des aktiven implantierbaren Medizinprodukts, umfassend mindestens eine Elektrode;

Figuren 2A bis 2D    jeweils eine schematische Darstellung einer bevorzugten Ausführung der Elektrode; und

Figur 3A und 3B    jeweils eine schematische Darstellung eines bevorzugten Verfahrens zur Herstellung des aktiven implantierbaren Medizinprodukts.

Beschreibung der Ausführungsbeispiele

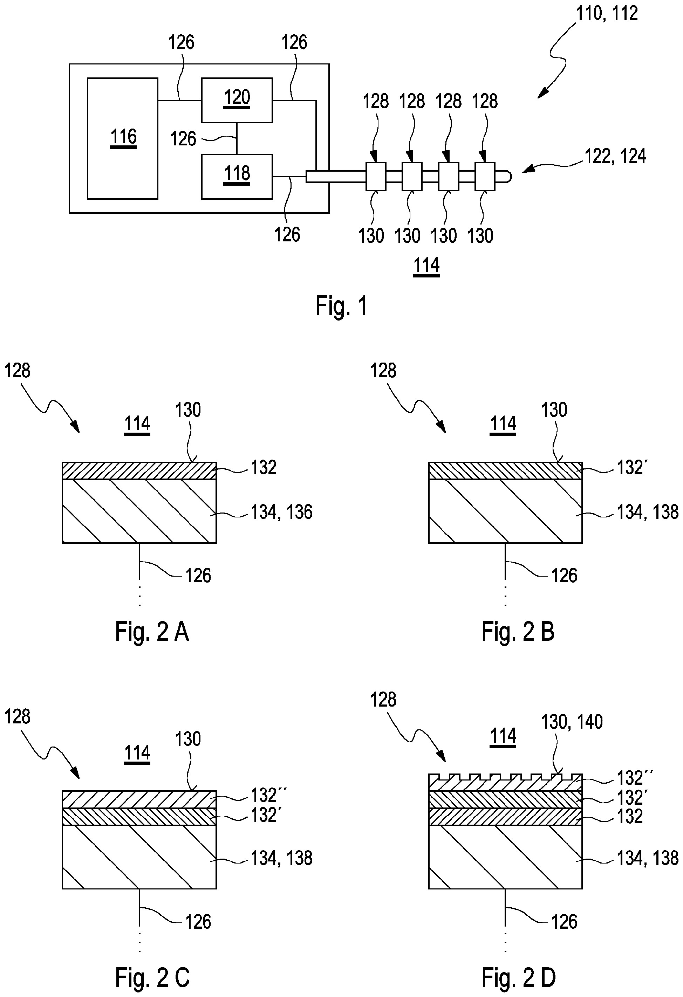

Figur 1 zeigt eine schematische Darstellung einer bevorzugten Ausführung eines aktiven implantierbaren Medizinprodukts 110 am Beispiel eines eigentlichen Cochlea-Implantats 112 zur Behandlung eines menschlichen oder tierischen Körpers, insbesondere zur Stimulation einer Gewebeumgebung 114 im Körper. Im Falle des Cochlea-Implantats 112 umfasst die zu stimulierende Gewebeumgebung 114 einen Hörnerv und/oder Ganglien der Hörbahn.

Das in Figur 1 schematisch dargestellte Ausführungsbeispiel für das Cochlea-Implantat 112 umfasst einen Schallsensor 116 zur Aufnahme eines Schallsignals und zur Umwandlung des Schallsignals in ein elektronisches Audiosignal, eine elektronische Signalverarbeitungseinheit 118 zur Verarbeitung und Verstärkung des elektronischen Audiosignals und eine Energiequelle 120 zur Energieversorgung des Schallsensors 116, der elektronischen Signalverarbeitungseinheit 118 sowie mindestens eines Aktors 122 zur Stimulation der Gewebeumgebung 114 im Körper, wobei der Aktor 122 hier beispielhaft in Form einer Ringelektrode 124 ausgeführt ist. Zur Energieversorgung besteht jeweils eine Anbindung 126 des Schallsensors 116 an die Energiequelle 120, der elektronischen Signalverarbeitungseinheit 118 an die Energiequelle 120 sowie der Ringelektrode 124 an die Energiequelle 120. Die hier beispielhaft dargestellte Ringelektrode 124 umfasst eine Mehrzahl an Elektroden 128, die jeweils eine an die Gewebeumgebung 114 zugewandte Oberfläche 130 aufweisen, durch welche eine gewünschte Stimulation der Gewebeumgebung 114 mittels der elektrischen Audiosignale erfolgen kann.

Die Figuren 2A bis 2D zeigen jeweils eine schematische, nicht-maßstäbliche Darstellung einer bevorzugten Ausführung der Elektrode 128 und der jeweiligen, der Gewebeumgebung 114 zugewandten Oberfläche 130 der Elektrode 128 in Form von Schnitten durch die betreffende Elektrode 128. Erfindungsgemäß weist die Elektrode 128 an der der Gewebeumgebung 114 zugewandten Oberfläche der Elektrode 128 eine biokompatible, unter Stimulationsbedingungen chemisch stabile Schicht aus mindestens einer metallhaltigen Verbindung auf, die in den vorliegenden Ausführungsbeispielen ohne Beschränkung der Allgemeinheit als Metalloxidschicht 132 ausgeführt ist. Für mögliche Alternativen zur Metalloxidschicht 132 wird auf die obige Beschreibung verwiesen.

In der bevorzugten Ausführung gemäß Figur 2A verfügt die Elektrode 128 über ein Substrat 134, das ein metallisches Elektrodenmaterial 136 umfasst, das bevorzugt ausgewählt ist aus der Gruppe enthaltend Titan, Zirkonium, Hafnium, Niob, Tantal, Molybdän, Wolfram und Rhodium. Die Metalloxidschicht 132 an der der Gewebeumgebung 114 zugewandten Oberfläche 130 der Elektrode 128 weist dagegen mindestens ein Metalloxid auf, das vorzugsweise mittels Anodisierung des ausgewählten metallischen Elektrodenmaterials 136 erzeugt wurde. Beispielsweise kann das Substrat 134 der Elektrode 128 eines der genannten Elektrodenmaterialien, insbesondere Rhodium, Tantal oder Niob, umfassen, während in der Metalloxidschicht 132 auf der der Gewebeumgebung 114 zugewandten Oberfläche 130 der Elektrode 128 Rhodiumoxid, Tantaloxid oder Nioboxid, ausgebildet ist. Alternativ ist hierbei auch die Ausbildung einer Schicht aus Tantalnitrid denkbar.

In experimentellen Untersuchungen konnte gezeigt werden, dass unter den spezifischen Stimulationsbedingungen für die Anregung der Gewebeumgebung 114 die Korrosion der Elektroden 128 im Vergleich zu einer Platinelektrode bei der Verwendung von Tantal um etwa $10^{-3}$ und in Niob um etwa $10^{-2}$ verringert werden konnte, während sowohl Tantal als auch Niob für eine positive Biokompatibilität bekannt sind. Die Verwendung eines anderen Materials oder eine Kombination von Materialien, insbesondere in Form einer metallischen Legierung, aus der oben genannten Liste der Elektrodenmaterialien 136 ist jedoch ebenfalls möglich.

In der weiterhin bevorzugten Ausführung gemäß Figur 2B verfügt die Elektrode 128 über ein Substrat 134, das ein metallisches Substratmaterial 138 umfasst, das bevorzugt ausgewählt ist aus der Gruppe enthaltend Ruthenium, Rhodium, Iridium, Gold, Stahl, Titan, Zirkonium, Hafnium, Niob und Tantal. Die Metalloxidschicht 132' kann hierbei durch ein additives Verfahren, insbesondere ausgewählt aus ALD, PVD oder CVD, auf das Substrat 134 aufgebracht werden. Hier kann insbesondere für das Substrat 134 in anderes Metall gewählt werden als das in der Metalloxidschicht 132 verwendete Metall.

In der weiterhin bevorzugten Ausführung gemäß Figur 2C verfügt die Elektrode 128 ebenfalls über ein Substrat 134, das ein metallisches Substratmaterial 138 gemäß der Beschreibung zu Figur 2B umfasst. Im Unterschied zu der Elektrode 128 gemäß Figur 2B, verfügt die Elektrode 128 gemäß Figur 2C zwei voneinander verschiedene biokompatible Metalloxidschichten 132', 132". Die Art der Ausführung der Elektrode 128 gemäß Figur 2C eröffnet eine Vielzahl von Möglichkeiten, die Elektrode 128 mit vorteilhaften Eigenschaften, insbesondere einer verstärkten mechanischen Stabilität oder einer erhöhten Flächenkapazität, auszustatten. Beispielsweise kann eine Tantaloxid- bzw. Nioboxidschicht der Gewebeumgebung 114 zugewandt sein. Andererseits kann eine zusätzliche Hafniumoxidschicht zu einer Erhöhung der Flächenkapazität der Elektrode 128 beitragen.

In der weiterhin bevorzugten Ausführung gemäß Figur 2D verfügt die Elektrode 128 ebenfalls über ein Substrat 134, das ein metallisches Substratmaterial 138 gemäß der Beschreibung zu Figur 2B umfasst. Im Unterschied zu der Elektrode 128 gemäß den Figuren 2B und 2C verfügt die Elektrode 128 gemäß Figur 2D drei voneinander verschiedene biokompatible Metalloxidschichten 132, 132', 132", wobei die beiden Metalloxidschichten 132', 132" wie in Figur 2C ausgeführt sein können, während die Metalloxidschicht 132 wie in Figur 2A ausgeführt ist, d.h. ein Metalloxid aufweist, das vorzugsweise mindestens einem Metalloxid des als das Substratmaterial 138 ausgewählten metallischen Elektrodenmaterials 136 entspricht.

In dieser Ausführung können die Metalle für die beiden Metalloxidschichten 132', 132" zunächst mittels eines additiven Verfahrens, insbesondere ausgewählt aus den oben näher bezeichneten Verfahren ALD, PVD oder CVD, auf das Substrat 134 aufgebracht werden, wobei hier die anschließende Anodisierung derart durchgeführt werden kann, dass sowohl die beiden Metalloxidschichten 132', 132" aus den auf das Substrat 134 aufgebrachten metallischen Materialien als auch die weitere Metalloxidschicht 132 aus dem metallischen Substratmaterial 138 erzeugt werden.

Beispielsweise kann das Substratmaterial 138 Titan umfassen, auf das eine Zirkoniumschicht oder eine Hafniumschicht aufgebracht wird, wobei anschließend eine Aufbringung einer Schicht aus Tantal oder Niob auf die Zirkoniumschicht oder auf die Hafniumschicht erfolgt. Zusätzlich kann jeweils eine Haftvermittlerschicht (nicht dargestellt) aufgebracht werden, bevor die Aufbringung der nächsten Schicht erfolgt. Die Durchführung der Anodisierung erfolgt derart, dass sowohl die Tantal- bzw. Niobschicht und die Zirkoniumschicht oder die Hafniumschicht als auch ein der Zirkoniumschicht oder der Hafniumschicht zugewandter Bereich des Substrats 134 aus Titan in ein jeweils zugehöriges Metalloxid umgewandelt wird. In dieser Anordnung kann die der Gewebeumgebung 114 zugewandte Tantaloxid- bzw. Nioboxidschicht der Elektrode 128 die Gewebeumgebung 114 vor einem direkten Kontakt mit den darunterliegenden Metalloxidschichten 132, 132' und insbesondere dem Substratmaterial 138 schützen. Andererseits kann die Zirkoniumschicht oder die Hafniumschicht zu einer Erhöhung der Flächenkapazität der Elektrode 128 beitragen, während die Schicht aus Titan für eine hohe mechanische Stabilität des Substrats 134 und damit der gesamten Elektrode 128 dienen kann. Andere Arten von Schichten sind jedoch möglich.

Weiterhin zeigt die in Figur 2D schematisch dargestellte Oberfläche 130 der oberen Metalloxidschicht 132" eine von einer planaren Ebene abweichende, ein Profil aufweisende Oberflächentopographie 140. Hierzu wurde die Oberfläche 130 der oberen Metalloxidschicht 132" mit einer periodischen Struktur versehen, die über eine charakteristische Länge, die in Beziehung zu einer Periode der Struktur steht, insbesondere einen Abstand zwischen zwei wiederkehrenden Höhen des Profils, verfügen kann. Die Oberflächentopographie 140 kann vorzugsweise mittels eines subtraktiven Verfahrens in die Oberfläche 130 der oberen Metalloxidschicht 132" eingebracht werden. Die Oberflächentopographie 140 kann insbesondere bewirken, dass die damit ausgestattete Elektrode 128 über eine höhere effektive Oberfläche 130 und damit eine höhere Flächenkapazität verfügen kann. Auf diese Weise kann eine Verringerung der Kapazität in Folge der erfindungsgemäß für die Elektroden verwendeten Metalloxide im Vergleich zu dem bisher verwendeten Platinoxid zumindest teilweise kompensiert werden.

Alternativ oder zusätzlich sind weitere Ausführungen der Elektrode 128 und insbesondere der jeweiligen, der Gewebeumgebung 114 zugewandten Oberfläche 130 der Elektrode 128 zusätzlich zu den in den Figuren 2A bis 2C dargestellten Ausführungsbeispielen denkbar. Insbesondere können die Elektroden 128 in den Figuren 2A bis 2C ebenso dieselbe oder eine andere Oberflächentopographie 140 gemäß Figur 2D in der jeweils oberen Metalloxidschicht 132, 132', 132" aufweisen.

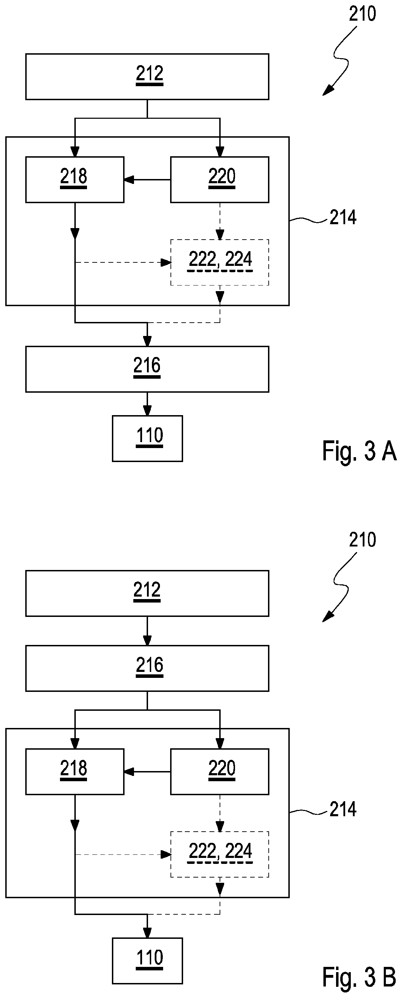

Die Figuren 3A und 3B zeigen jeweils eine schematische Darstellung eines bevorzugten Verfahrens 210 zur Herstellung des aktiven implantierbaren Medizinprodukts 110.

Wie in beiden Figuren 3A und 3B dargestellt, erfolgt gemäß einem Bereitstellungsschritt 212 ein Bereitstellen der mindestens einen elektrisch leitfähigen Elektrode 128.

Hieran anschließend erfolgt gemäß einem Behandlungsschritt 214 ein Behandeln der Oberfläche 130 der Elektrode 128 in einer Weise, dass hierdurch die gewünschte biokompatible, unter den Stimulationsbedingungen chemisch stabile Metalloxidschicht 132 ausgebildet wird.

Wie Figur 3A zeigt, kann hieran anschließend ein Anbindungsschritt 216 ausgeführt werden, gemäß dem ein Herstellen der mindestens einen Anbindung 126 der mindestens einen Elektrode 128 an die mindestens eine Energiequelle 120 im Anschluss an den Behandlungsschritt 214 erfolgt. Beispielsweise kann die mindestens eine Elektrode 218 mittels Kontaktschweißen auf der der Gewebeumgebung 114 abgewandten Rückseite der Elektrode 218 mit der mindestens einen Anbindung 126 versehen werden.

Im Unterschied hierzu kann gemäß der Darstellung in Figur 3B der Anbindungsschritt 216 bereits vor dem Behandlungsschritt 214 ausgeführt werden. Es ist zu erwarten, dass auf diese Weise eine verbesserte Ausführung einer Auflösung eines Randes der Elektrode 128 erreicht werden kann.

Nach Abschluss der Durchführung sowohl des Behandlungsschritts 214 als auch des Anbindungsschritts 216 kann die mit der mindestens einen Anbindung 126 versehene Elektrode 128 in eine dafür vorgesehene Form eingelegt und mit einem inerten Material, beispielsweise Silikon, bedeckt werden. Damit kann die mindestens eine Anbindung 126 in vorteilhafter Weise derart verpackt werden, dass das sich unter dem inerten Material in der Elektrode 128 befindende Metall vor einer Auflösung in der Körperflüssigkeit geschützt werden kann.

Wie weiterhin aus beiden Figuren 3A und 3B hervorgeht, kann der Behandlungsschritt 214, insbesondere im Hinblick auf die verwendeten Elektrodenmaterialien 136 und Substratmaterialien 138, auf unterschiedliche Weise ausgeführt werden.

In einer bevorzugten Ausführung des Behandlungsschritts 214 kann, wie in Figur 2A schematisch dargestellt, die Metalloxidschicht 132 an der der Gewebeumgebung 114 zugewandten Oberfläche 130 der Elektrode 128 mittels Anodisierung 218 des ausgewählten metallischen Elektrodenmaterials 136 erzeugt werden, wobei das metallische Elektrodenmaterial 136 bevorzugt ausgewählt ist aus der Gruppe enthaltend Titan, Zirkonium, Hafnium, Niob, Tantal, Molybdän, Wolfram und Rhodium.

Wie bereits erwähnt, kann die Anodisierung 218 bei einer Anodisierungsspannung erfolgen, welche eine gemäß den Stimulationsbedingungen zum Beaufschlagen der der Metalloxidschicht 132 zugewandten Gewebeumgebung 114 festgelegte elektrische Spannung übersteigt. Zur Durchführung der Anodisierung 218 kann daher es besonders vorteilhaft sein, wenn das Metalloxid eine ausreichende elektrische Festigkeit, insbesondere eine hohe dielektrische Durchbruchsfestigkeit unter Stimulationsbedingungen aufweist. Zu diesem Zweck, kann als Anodisierungsspannung vorzugsweise ein Wert gewählt werden, welcher einem Faktor von mindestens 1,25, bevorzugt von mindestens 1,5, besonders bevorzugt von mindestens 2, der bei der Anwendung des aktiven implantierbaren Medizinprodukts zu erwartenden maximalen Stimulationsspannung $U_{max}$ liegt. Die heute benutzte maximale Stimulationsspannung $U_{max}$ liegt bei 7 V bis 8 V, zukünftig werden jedoch Werte für $U_{max}$ von 12 bis 14 V erwartet. Hierbei kann für eine durch die Anodisierung 218 erreichbare sichere Schichtdicke der Metalloxidschicht 132 anhand eines aus G.E. Thompson, H. Habazaki, K. Shimizu et. al. Anodic film growth on tantalum in dilute phosphoric acid solution at 20 and 85°C, Electrochimica Acta, 47, 2002, S. 2761-2767, bekannten Schichtbildungsfaktors von 1,67 nm/V für Tantal in verdünnter Phosphorsäure bei 5 mA cm-2 und 20°C aus diesen Spannungen gemäß der folgenden **Tabelle** bestimmt werden:

| Schichtdicke | $U_{max}$ = 7 V | $U_{max}$ = 12 V |
|---|---|---|
| Faktor 1,5 | 17,5nm | 30,1 nm |
| Faktor 2 | 23,4 nm | 401 nm |

Wie in P.J. Boyle, Electrical Stimulation of the Auditory System, May 31, 2019, IntechOpen, DOI: 10.5772/intechopen.85285, erhältlich über https://www.intechopen.com/online-first/ electrical-stimulation-of-the-auditory-system, beschrieben, wird in üblichen Stimulationselektroden ein maximaler Strom von 2 mA abgegeben; die Elektroden haben eine Impedanz von mehreren kΩ und es wird eine maximale Ladungsdichte von 30 $\mu$C/cm$^2$ bei biphasischen Pulsen mit einer Pulsweite von 40-50 $\mu$sec eingesetzt, d.h. eine Pulsweite 20-25 $\mu$sec pro Phase (anodisch-kathodisch). Die maximal abgegebene Spannung liegt hier bei 8 V, in Zukunft werden 14 V erwartet.

Entsprechend lässt sich folgern, dass bei Abmessungen der Stimulationselektroden von 400 $\mu$m · 400 $\mu$m, dies einer Fläche von 1,6·10$^{-3}$ cm$^2$ entspricht, wobei unter Verwendung der maximale Ladungsdichte von 30 $\mu$C/cm$^2$ ein Wert von 48 nC für die elektrische Ladung Q erhalten wird, der von den Elektroden maximal übertragen werden. Unter Benutzung der bekannten Beziehung für die elektrische Ladung Q = Kapazität C · Spannung U, wobei die Spannung U = 8 V eingesetzt wird, kann ermittelt werden, dass die errechnete Ladung Q = 48 nC eine Kapazität C = 6 nF erfordert. Unter Verwendung der ebenfalls bekannten Beziehung für die Kapazität eines plattenförmigen Kondensators C = $\varepsilon_0$ · $\varepsilon_r$ · A / d, wobei $\varepsilon_0$ die elektrische Feldkonstante, $\varepsilon_r \approx$ 10 die relative Permittivität von Platinoxid, A die Fläche des Kondensators und d die gesuchte Schichtdicke angeben, kann eine maximale Schichtdicke von Platinoxid d $\approx$ 2,3 nm ermittelt werden.

Wird nun ein Metall gemäß der vorgeschlagenen Erfindung eingesetzt, so ist es vorteilhaft, möglichst dieselbe Stromtragfähigkeit wie in bisherigen Implantaten zu erreichen. Wird insbesondere aus Sicherheitsgründen eine höhere Anodisierungsspannung als die maximal verwendeten Stimulationsspannungen eingesetzt, so kann eine Schichtdicke der Metalloxidschicht von 1 nm/V bis 2 nm/V, insbesondere 1,45 nm/V angenommen werden. Bei einem Sicherheitsfaktor von 2, kann die Anodisierung somit bei einer Anodisierungsspannung von mindestens 1,6 V, in Zukunft von mindestens 2,8 V, erfolgen. Die entsprechende Schichtdicke der Metalloxidschicht läge damit bei d $\approx$ 0,24 nm bzw. bei d $\approx$ 0,42 nm, mit Sicherheitsfaktor bei 1 nm bis 2 nm.

Für bevorzugte Parameter zur Durchführung der Anodisierung 218 wird auf die obige Beschreibung verwiesen. In einer besonders vorteilhaften Ausführung kann die Anodisierung 218 in einem Elektrolyten erfolgen, der mindestens einen Puffer umfasst, wobei der Puffer ausgewählt ist aus einem Acetatpuffer oder einem Citratpuffer. Acetatpuffer, Citratpuffer und ihre Spuren sind biokompatibel und ermöglichen eine Durchführung der Anodisierung 218 in neutralen Lösungen, ohne dass ein Einsatz von Fluorid-Ionen oder Chlorid-Ionen, die als Komplexbildner wirken und in üblichen Anodisierungsmedien wie HCl oder H$_2$SO$_4$ auftreten, oder von auch Borsäure, die wegen möglicher Borspuren im Metalloxid nachteilig ist, erforderlich wäre.

In einer weiterhin bevorzugten Ausführung des Behandlungsschritts 214 kann die in Figur 2B und 2C schematisch dargestellte, mindestens eine Metalloxidschicht 132', 132" mittels eines additiven Verfahrens 220, das insbesondere ausgewählt ist aus den oben näher ALD, PVD oder CVD, auf das Substrat 134 aufgebracht werden. In dieser Ausführung kann die Elektrode 128 ein Substrat 134 ausweisen, das ein metallisches Substratmaterial 138 umfasst, das bevorzugt ausgewählt ist aus der Gruppe enthaltend Gold, Stahl, Titan, Zirkonium, Hafnium, Niob, Tantal, Ruthenium, Rhodium und Iridium. Vorteilhaft an dieser Ausführung ist, dass hierbei insbesondere unterschiedliche Metalle für das Substrat 134 und die mindestens eine Metalloxidschicht 132', 132" gewählt werden können.

In einer bevorzugten Ausführung des Behandlungsschritts 214 kann die in Figur 2D schematisch dargestellte, mindestens eine Metallschicht mittels des additiven Verfahrens 220, insbesondere ausgewählt aus den oben näher bezeichneten Verfahren ALD, PVD oder CVD, auf das Substrat 134 aufgebracht werden, wobei anschließend die mindestens eine Metallschicht mittels der Anodisierung 218 derart behandelt wird, dass zumindest die mindestens eine Metalloxidschicht 132', 132" aus den auf das Substrat 134 aufgebrachten Metallschichten erzeugt wird. Je nach Art der Durchführung der Anodisierung kann zusätzlich auch die weitere Metalloxidschicht 132 aus dem metallischen Substratmaterial 138 selbst erzeugt werden.

Beispielsweise kann das Substrat 134 Titan umfassen, auf das mittels des additiven Verfahrens 220 zunächst eine Hafniumschicht und anschließend auf diese eine Tantal- oder Niobschicht aufgebracht wird. Die Durchführung der Anodisierung 218 kann derart erfolgen, dass zumindest die Tantal- bzw. Niobschicht und die Hafniumschicht, bevorzugt aber auch ein der Hafniumschicht zugewandter Bereich des Substrats 134 aus Titan in das jeweilige Metalloxid umgewandelt wird. Andere Arten von Schichten sind jedoch möglich.

Zusätzlich kann jeweils eine Haftvermittlerschicht (nicht dargestellt) aufgebracht werden, bevor die Aufbringung der nächsten Schicht erfolgt.

Der Behandlungsschritt 214 kann in einer bevorzugten Ausgestaltung darüber hinaus einen Profilierungsschritt 222 umfassen, der zur Erzeugung einer von einer planaren Ebene abweichenden, ein Profil aufweisenden Oberflächentopographie 140 auf der Oberfläche 130 der oberen Metalloxidschicht 132" eingerichtet ist. Wie bereits erwähnt, kann die Oberflächentopographie 140 hierbei vorzugsweise mittels eines subtraktiven Verfahrens 224 in die Oberfläche 130 der oberen Metalloxidschicht 132" eingebracht werden. Andere Verfahren zur Erzeugung der Oberflächentopographie 140 sind jedoch möglich.

In einer besonders bevorzugten Ausführung (nicht dargestellt) kann während des Bereitstellungsschritts 212 gleichzeitig eine Vielzahl an Elektroden in Form eines gemeinsamen Substrats bereitgestellt und jeweils in einem Anbindungsschritt 216 mit der Anbindung 126 versehen werden, bevor hieran anschließend der Behandlungsschritt 214 für die Vielzahl der Elektroden in dem gemeinsamen Substrat erfolgt. In dieser Ausführung kann insbesondere der Einsatz des additiven Verfahrens 220 zur Erzeugung einer gemeinsamen Metalloxidschicht von Vorteil sein. Abschließend kann dann eine Abtrennung der einzelnen Elektroden 218 aus dem gemeinsamen Substrat erfolgen, wobei jede einzelne Elektrode 218 dann eine eigene Metalloxidschicht 132 aufweisen kann.

Bezugszeichenliste

| | |
|---|---|
| 110 | aktives implantierbares Medizinprodukt |
| 112 | Cochlea-Implantat |
| 114 | Gewebeumgebung |
| 116 | Schallsensor |
| 118 | elektronische Signalverarbeitungseinheit |
| 120 | Energiequelle |
| 122 | Aktor |
| 124 | Ringelektrode |
| 126 | Anbindung |
| 128 | Elektrode |
| 130 | Oberfläche |
| 132, 132', 132" | Schicht einer metallhaltigen Verbindung, insbesondere Metalloxidschicht |
| 134 | Substrat |
| 136 | Elektrodenmaterial |
| 138 | Substratmaterial |
| 140 | Oberflächentopographie |
| 210 | Verfahren zur Herstellung eines aktiven implantierbaren Medizinprodukts |
| 212 | Bereitstellungsschritt |
| 214 | Behandlungsschritt |
| 216 | Anbindungsschritt |
| 218 | Anodisierung |
| 220 | additives Verfahren |
| 222 | Profilierungsschritt |
| 224 | subtraktives Verfahren |

**Patentansprüche**

1. Aktives implantierbares Medizinprodukt (110) zur Behandlung eines menschlichen oder tierischen Körpers, umfassend

   - mindestens eine elektrisch leitfähige Elektrode (128) zur Stimulation einer zumindest teilweise einer Oberfläche (130) der Elektrode (128) zugewandten Gewebeumgebung (114) im Körper, wobei die mindestens eine Elektrode (128) zumindest ein Elektrodenmaterial (136) aufweist;
   - mindestens eine Energiequelle (120) zur Bereitstellung von Energie zur Stimulation der Gewebeumgebung (114); und
   - mindestens eine Anbindung (126) der mindestens einen Elektrode (128) an die mindestens eine Energiequelle (120),

   wobei die mindestens eine Elektrode (128) ferner an der der Gewebeumgebung (114) zugewandten Oberfläche (130) der mindestens einen Elektrode (128) eine biokompatible, unter Stimulationsbedingungen chemisch stabile Schicht (132) mindestens einer metallhaltigen Verbindung aufweist, **dadurch gekennzeichnet, dass** die Schicht (132) der metallhaltigen Verbindung elektrisch isolierend ist.

2. Aktives implantierbares Medizinprodukt (110) nach dem vorangehenden Anspruch, wobei die Schicht (132) der metallhaltigen Verbindung mindestens ein Oxid, ein Hydroxid, ein Nitrid, ein Karbid, ein Oxykarbid oder eine Kombination hiervon des Elektrodenmaterials (136) aufweist.

3. Aktives implantierbares Medizinprodukt (110) nach einem der vorangehenden Ansprüche, wobei das Elektrodenmaterial (136) ausgewählt ist aus der Gruppe enthaltend Titan, Zirkonium, Hafnium, Niob, Tantal, Iridium und Rhodium.

4. Aktives implantierbares Medizinprodukt (110) nach einem der vorangehenden Ansprüche, wobei die Schicht (132) der metallhaltigen Verbindung in Form mindestens einer Schicht auf einem Substrat (134) aufgebracht ist.

5. Aktives implantierbares Medizinprodukt (110) nach dem vorangehenden Anspruch, wobei das Substrat (134) mindestens ein Substratmaterial (138) aufweist, wobei das Substratmaterial (138) ausgewählt ist aus der Gruppe enthaltend Gold, Stahl, Titan, Zirkonium, Hafnium, Niob, Tantal, Ruthenium, Rhodium und Iridium.

6. Aktives implantierbares Medizinprodukt (110) nach einem der vorangehenden Ansprüche, wobei die Stimulationsbedingungen ein Beaufschlagen der der Oberfläche (130) der Schicht (132) der metallhaltigen Verbindung zugewandten Gewebeumgebung (114) im Körper mit einer von der Gewebeumgebung (114) abhängigen elektrischen Spannung umfassen.

7. Aktives implantierbares Medizinprodukt (110) nach einem der vorangehenden Ansprüche, wobei die Oberfläche (130) der Schicht (132) der metallhaltigen Verbindung eine von einer planaren Ebene abweichende Oberflächentopographie (140) aufweist.

8. Verfahren (210) zur Herstellung eines aktiven implantierbaren Medizinprodukts (110) zur Behandlung eines menschlichen oder tierischen Körpers, umfassend die Verfahrensschritte:

   a) Bereitstellen mindestens einer elektrisch leitfähigen Elektrode (128);
   b) Behandeln der Oberfläche (130) der Elektrode (128), wobei die biokompatible, unter Stimulationsbedingungen chemisch stabile Schicht (132) mindestens einer metallhaltigen Verbindung ausgebildet wird, wobei die Schicht (132) der metallhaltigen Verbindung elektrisch isolierend ist; und

c) Herstellen mindestens einer Anbindung (126) der mindestens einen Elektrode (128) an die mindestens eine Energiequelle (120) zur Bereitstellung von Energie zur Stimulation einer zumindest teilweise der Schicht (132) der mindestens einen metallhaltigen Verbindung zugewandten Gewebeumgebung (114) im Körper.

9. Verfahren (210) nach dem vorangehenden Anspruch, wobei das Behandeln der Oberfläche (130) der Elektrode (132) gemäß Schritt b) eine Anodisierung (218) eines Teils des Elektrodenmaterials (136) umfasst.

10. Verfahren (210) nach dem vorangehenden Anspruch, wobei die Anodisierung (218) bei einer Anodisierungsspannung erfolgt, die eine gemäß den Stimulationsbedingungen zum Beaufschlagen der der Schicht (132) der metallhaltigen Verbindung zugewandten Gewebeumgebung (114) im Körper festgelegte elektrische Spannung übersteigt.

11. Verfahren (210) nach einem der beiden vorangehenden Ansprüche, wobei die Anodisierung (218) des Elektrodenmaterials (136) bei einer Rampe der Anodisierung von 10 mV/s bis 100 mV/s erfolgt, wobei eine Haltezeit von 100 s bis 10000 s bei einem Maximum der Anodisierungsspannung eingehalten wird, und wobei das Elektrodenmaterial (136) mit Spannungspulsen von 10 V bis 100 V oberhalb der Anodisierungsspannung während mindestens einer Zeitdauer von 0,1 ms bis 10 ms beaufschlagt wird.

12. Verfahren (210) nach einem der drei vorangehenden Ansprüche, wobei die Anodisierung (218) in einem Elektrolyten erfolgt, wobei der Elektrolyt mindestens einen Puffer umfasst, wobei der Puffer ausgewählt ist aus einem Acetatpuffer oder einem Citratpuffer.

13. Verfahren (210) nach einem der vorangehenden Verfahrensansprüche, wobei das Behandeln der Oberfläche (130) der Elektrode (128) gemäß Schritt b) ein Aufbringen der mindestens einen Schicht (132) der metallhaltigen Verbindung auf ein Substrat (134) oder das Aufbringen mindestens einer Metallschicht auf das Substrat (134) mit anschließender Anodisierung (218) der mindestens einen Metallschicht umfasst.

14. Verfahren (210) nach einem der vorangehenden Verfahrensansprüche, wobei das Herstellen der mindestens einen Anbindung (126) gemäß Schritt c) vor oder nach dem Behandeln der Oberfläche (130) der Elektrode (128) gemäß Schritt b) erfolgt.

## Claims

1. Active implantable medical product (110) for treating a human or animal body, comprising

    - at least one electrically conductive electrode (128) for stimulating a surrounding tissue (114) in the body, at least part of which surrounding tissue faces a surface (130) of the electrode (128), wherein the at least one electrode (128) comprises at least one electrode material (136);
    - at least one energy source (120) for providing energy to stimulate the surrounding tissue (114); and
    - at least one connection (126) between the at least one electrode (128) and the at least one energy source (120),

    wherein the at least one electrode (128) furthermore comprises, on the surface (130) of the at least one electrode (128) facing the surrounding tissue (114), a biocompatible and, under stimulation conditions, chemically stable layer (132) of at least one metal-containing compound, **characterized in that** the layer (132) of metal-containing compound is electrically insulating.

2. Active implantable medical product (110) according to the preceding claim, wherein the layer (132) of metal-containing compound comprises at least one oxide, a hydroxide, a nitride, a carbide, an oxycarbide, or a combination thereof, of the electrode material (136).

3. Active implantable medical product (110) according to either of the preceding claims, wherein the electrode material (136) is selected from the group containing titanium, zirconium, hafnium, niobium, tantalum, iridium and rhodium.

4. Active implantable medical product (110) according to one of the preceding claims, wherein the layer (132) of metal-containing compound is applied in the form of at least one layer to a substrate (134).

5. Active implantable medical product (110) according to the preceding claim, wherein the substrate (134) comprises at least one substrate material (138), wherein the substrate material (138) is selected from the group containing gold, steel, titanium, zirconium, hafnium, niobium, tantalum, ruthenium, rhodium and iridium.

6. Active implantable medical product (110) according to one of the preceding claims, wherein the stimulation conditions include applying an electrical voltage, which is dependent on the surrounding tissue (114), to the surrounding tissue (114) in the body that faces the surface (130) of the layer (132) of metal-containing compound.

7. Active implantable medical product (110) according to one of the preceding claims, wherein the surface (130) of the layer (132) of metal-containing compound has a surface topography (140) other than a planar plane.

8. Method (210) for producing an active implantable medical product (110) for treating a human or animal body, comprising the following method steps:

   a) providing at least one electrically conductive electrode (128);
   b) treating the surface (130) of the electrode (128), wherein the biocompatible and, under stimulation conditions, chemically stable layer (132) of at least one metal-containing compound is formed, wherein the layer (132) of metal-containing compound is electrically insulating; and
   c) establishing at least one connection (126) between the at least one electrode (128) and the at least one energy source (120) to provide energy for stimulating a surrounding tissue (114) in the body, at least part of which surrounding tissue faces the layer (132) of at least one metal-containing compound.

9. Method (210) according to the preceding claim, wherein the treatment of the surface (130) of the electrode (132) according to step b) includes anodizing (218) a portion of the electrode material (136).

10. Method (210) according to the preceding claim, wherein the anodization (218) is performed at an anodizing voltage that exceeds an electrical voltage that is stipulated according to the stimulation conditions for applying to the surrounding tissue (114) in the body, which surrounding tissue faces the layer (132) of metal-containing compound.

11. Method (210) according to one of the two preceding claims, wherein the electrode material (136) is anodized (218) at an anodization ramp of 10 mV/s to 100 mV/s, wherein a holding time of 100 s to 10000 s at a maximum of the anodizing voltage is observed, and wherein voltage pulses of 10 V to 100 V above the anodizing voltage are applied to the electrode material (136) over at least a period of 0.1 ms to 10 ms.

12. Method (210) according to one of the three preceding claims, wherein the anodization (218) takes places in an electrolyte, wherein the electrolyte comprises at least one buffer, wherein the buffer is selected from an acetate buffer or a citrate buffer.

13. Method (210) according to one of the preceding method claims, wherein the treatment of the surface (130) of the electrode (128) according to step b) includes applying the at least one layer (132) of metal-containing compound to a substrate (134) or applying at least one metal layer to the substrate (134) with subsequent anodization (218) of the at least one metal layer.

14. Method (210) according to one of the preceding method claims, wherein the at least one connection (126) according to step c) is produced before or after the surface (130) of the electrode (128) is treated according to step b).

**Revendications**

1. Produit médical implantable actif (110) destiné au traitement d'un corps humain ou animal, comprenant

   - au moins une électrode électriquement conductrice (128) destinée à stimuler un tissu environnant (114) dans le corps, tourné au moins partiellement vers une surface (130) de l'électrode (128), ladite au moins une électrode (128) présentant au moins un matériau d'électrode (136) ;
   - au moins une source d'énergie (120) destinée à fournir de l'énergie pour stimuler le tissu environnant (114) ; et
   - au moins une liaison (126) de ladite au moins une électrode (128) à ladite au moins une source d'énergie (120),

   dans lequel ladite au moins une électrode (128) présente en outre sur la surface (130), tournée vers le tissu environnant (114), de ladite au moins électrode (128) une couche biocompatible (132), chimiquement stable dans des conditions de stimulation, d'au moins un composé contenant du métal, **caractérisé en ce que** la couche (132) du composé contenant du métal est électriquement isolante.

2. Produit médical implantable actif (110) selon la revendication précédente, dans lequel la couche (132) du composé contenant du métal présente au moins un oxyde, un hydroxyde, un nitrure, un carbure, un oxycarbure ou une combinaison de ceux-ci du matériau d'électrode (136).

3. Produit médical implantable actif (110) selon l'une quelconque des revendications précédentes, dans lequel le matériau d'électrode (136) est sélectionné dans le groupe contenant du titane, du zirconium, du hafnium, du niobium, du tantale, de l'iridium et du rhodium.

4. Produit médical implantable actif (110) selon l'une quelconque des revendications précédentes, dans lequel la couche (132) du composé contenant du métal est appliquée sur un substrat (134) sous la forme d'au moins une couche.

5. Produit médical implantable actif (110) selon la revendication précédente, dans lequel le substrat (134) présente au moins un matériau de substrat (138), dans lequel le matériau de substrat (138) est sélectionné dans le groupe contenant de l'or, de l'acier, du titane, du zirconium, du hafnium, du niobium, du tantale, du ruthénium, du rhodium et de l'iridium.

6. Produit médical implantable actif (110) selon l'une quelconque des revendications précédentes, dans lequel les conditions de stimulation comprennent une sollicitation du tissu environnant (114) dans le corps, tourné vers la surface (130) de la couche (132) du composé contenant du métal, avec une tension électrique dépendant du tissu environnant (114).

7. Produit médical implantable actif (110) selon l'une quelconque des revendications précédentes, dans lequel la surface (130) de la couche (132) du composé contenant du métal présente une topographie de surface (140) différente d'un plan planaire.

8. Procédé (210) permettant de fabriquer un produit médical (110) implantable actif destiné à traiter un corps humain ou animal, comprenant les étapes de procédé consistant à :

a) fournir au moins une électrode (128) électriquement conductrice ;
b) traiter la surface (130) de l'électrode (128), dans lequel la couche biocompatible (132), chimiquement stable dans des conditions de stimulation, d'au moins un composé contenant du métal est réalisée, la couche (132) du composé contenant du métal étant électriquement isolante ; et
c) fabriquer au moins une liaison (126) de ladite au moins une électrode (128) à ladite au moins une source d'énergie (120) pour fournir de l'énergie pour la stimulation d'au moins un tissu environnant (114) dans le corps, tourné au moins partiellement vers la couche (132) dudit au moins un composé contenant du métal.

9. Procédé (210) selon la revendication précédente, dans lequel le traitement de la surface (130) de l'électrode (132) selon l'étape b) comprend une anodisation (218) d'une partie du matériau d'électrode (136).

10. Procédé (210) selon la revendication précédente, dans lequel l'anodisation (218) est effectuée à une tension d'anodisation qui dépasse une tension électrique définie selon les conditions de stimulation pour solliciter le tissu environnant (114) dans le corps, tourné vers la couche (132) du composé contenant du métal.

11. Procédé (210) selon l'une des deux revendications précédentes, dans lequel l'anodisation (218) du matériau d'électrode (136) est effectuée à une rampe de l'anodisation de 10 mV/s à 100 mV/s, dans lequel un temps de maintien de 100 s à 10 000 s est respecté à un maximum de la tension d'anodisation, et dans lequel le matériau d'électrode (136) est soumis à des impulsions de tension de 10 V à 100 V au-dessus de la tension d'anodisation pendant au moins une durée de 0,1 ms à 10 ms.

12. Procédé (210) selon l'une des trois revendications précédentes, dans lequel l'anodisation (218) est effectuée dans un électrolyte, l'électrolyte comprenant au moins une solution tampon, la solution tampon étant sélectionnée parmi une solution tampon d'acétate ou une solution tampon de citrate.

13. Procédé (210) selon l'une quelconque des revendications de procédé précédentes, dans lequel le traitement de la surface (130) de l'électrode (128) selon l'étape b) comprend une application de ladite au moins une couche (132) du composé contenant du métal à un substrat (134) ou l'application d'au moins une couche de métal au substrat (134) avec une anodisation consécutive (218) de ladite au moins une couche de métal.

14. Procédé (210) selon l'une quelconque des revendications de procédé précédentes, dans lequel la fabrication de ladite au moins une liaison (126) selon l'étape c) est effectuée avant ou après le traitement de la surface (130) de l'électrode (128) selon l'étape b).

Fig. 1

Fig. 2 A

Fig. 2 B

Fig. 2 C

Fig. 2 D

Fig. 3 A

Fig. 3 B

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2019206664 A1
- EP 1421972 A2
- WO 2016130402 A1

**In der Beschreibung aufgeführte Nicht-Patentliteratur**

- **A. W. HASSEL** ; **J. P. KOLLENDER** ; **G. SPRINZL** ; **T. DOLL**. Corrosion of active implant materials for cochlear hearing aids and cardiac pacemakers. *EUROCORR 2017 & 20th ICC Prag*, 03 September 2017
- **CLARK, GRAEME**. Cochlear Implants: Fundamentals and Applications. Springer, 2003, 171
- **G.E. THOMPSON** ; **H. HABAZAKI** ; **K. SHIMIZU**. Anodic film growth on tantalum in dilute phosphoric acid solution at 20 and 85°C. *Electrochimica Acta*, 2002, vol. 47, 2761-2767
- **P.J. BOYLE**. Electrical Stimulation of the Auditory System. IntechOpen, 31 May 2019